# EUROPEAN PATENT APPLICATION

(11) **EP 2 784 094 A1**
(43) Date of publication of application: **01.10.2014**
(21) Application number: 13305400.7
(22) Date of filing: 28.03.2013
(51) Int. Cl.: C07K 19/00, C07K 14/47

(54) **New uranium-chelating peptides derived from EF-hand calcium-binding motif useful for uranium biodetection and biodecontamination**

(71) Applicant: COMMISSARIAT A L'ENERGIE ATOMIQUE ET AUX ENERGIES ALTERNATIVES, 75015 Paris (FR)
(72) Inventor: Pardoux, Romain, 13080 Luynes (FR); Sauge-Merle, Sandrine, 84120 Pertuis (FR); Lemaire, David, 04100 Manosque (FR); Berthomieu, Catherine, 13100 Aix-en-Provence (FR); Guilbaud, Philippe, 30400 Villeneuve-lès-Avignon (FR); Delangle, Pascale, 38500 Voiron (FR); Bremond, Nicolas, 13100 Aix-en-Provence (FR); Beccia, Maria Rosa, 13100 Aix-en-Provence (FR)
(74) Representative: Leblois-Préhaud, Hélène Marthe Georgette

(57) **Abstract**

Uranium-chelating polypeptides comprising at least one helix-loop-helix calcium-binding (EF-hand) motif which comprises a deletion of at least one amino acid in the 12-amino-acid calcium-binding loop sequence, and their use for uranium biodetection and biodecontamination.

SEQUENCE LISTING

<110>  Commissariat à l'Energie Atomique et aux Energies
       Alternatives

<120>  New uranium-chelating peptides derived from EF-hand
       calcium-binding motif useful for uranium biodetection and
       biodecontamination

<130>  263EP558

<160>  54

<170>  PatentIn version 3.5

<210>  1
<211>  452
<212>  DNA
<213>  Arabidopsis thaliana

<400>  1
atggcggatc agctcaccga cgatcagatc tctgagttta aggaagcttt cagcttattc      60

gacaaggatg gtgatggttg cattaccacc aaggagctgg gtactgtgat gcgttccctt     120

ggacaaaacc caaccgaagc agagcttcaa gacatgatca acgaagtgga tgctgatggt     180

aacggtacca ttgatttccc agagttcttg aaccttatgg ctcgtaagat gaaggacacc     240

gactctgagg aagagctcaa ggaagcattc cgggttttcg acaaggacca gaacggtttc     300

atctcagcag ctgagctccg ccatgtgatg acaaaccttg gcgagaagct tactgatgaa     360

gaagttgatg agatgatcaa ggaagctgat gttgatggtg atggtcagat taactacgaa     420

gagtttgtta aggtcatgat ggctaagtga ct                                   452


<210>  2
<211>  149
<212>  PRT
<213>  Arabidopsis thaliana

<400>  2

Met Ala Asp Gln Leu Thr Asp Asp Gln Ile Ser Glu Phe Lys Glu Ala
1               5                   10                  15


Phe Ser Leu Phe Asp Lys Asp Gly Asp Gly Cys Ile Thr Thr Lys Glu
            20                  25                  30


Leu Gly Thr Val Met Arg Ser Leu Gly Gln Asn Pro Thr Glu Ala Glu
        35                  40                  45


Leu Gln Asp Met Ile Asn Glu Val Asp Ala Asp Gly Asn Gly Thr Ile
    50                  55                  60


Asp Phe Pro Glu Phe Leu Asn Leu Met Ala Arg Lys Met Lys Asp Thr
65                  70                  75                  80

Asp Ser Glu Glu Glu Leu Lys Glu Ala Phe Arg Val Phe Asp Lys Asp
                85                  90                  95

Gln Asn Gly Phe Ile Ser Ala Ala Glu Leu Arg His Val Met Thr Asn
            100                 105                 110

Leu Gly Glu Lys Leu Thr Asp Glu Glu Val Asp Glu Met Ile Lys Glu
        115                 120                 125

Ala Asp Val Asp Gly Asp Gly Gln Ile Asn Tyr Glu Glu Phe Val Lys
    130                 135                 140

Val Met Met Ala Lys
145

<210>  3
<211>  12
<212>  PRT
<213>  artificial sequence

<220>
<223>  synthetic peptide CaM3 EF-hand1 loop

<400>  3

Asp Lys Asp Gly Asp Gly Cys Ile Thr Thr Lys Glu
1               5                   10

<210>  4
<211>  12
<212>  PRT
<213>  artificial sequence

<220>
<223>  synthetic peptide CaM3 EF-hand2 loop

<400>  4

Asp Ala Asp Gly Asn Gly Thr Ile Asp Phe Pro Glu
1               5                   10

<210>  5
<211>  12
<212>  PRT
<213>  artificial sequence

<220>
<223>  synthetic peptide CaM3 EF-hand3 loop

<400>  5

Asp Lys Asp Gln Asn Gly Phe Ile Ser Ala Ala Glu
1               5                   10

<210>  6
<211>  12
<212>  PRT
<213>  artificial sequence

<220>
<223>  synthetic peptide CaM3 EF-hand4 loop

<400>  6

Asp Val Asp Gly Asp Gly Gln Ile Asn Tyr Glu Glu
1               5                   10


<210>  7
<211>  10
<212>  PRT
<213>  artificial sequence

<220>
<223>  synthetic peptide EF-hand1 loop delta-K2D3

<400>  7

Asp Gly Asp Gly Cys Ile Thr Thr Lys Glu
1               5                   10


<210>  8
<211>  10
<212>  PRT
<213>  artificial sequence

<220>
<223>  artificial sequence EF-hand1 loop delta-K2D3/C7Y

<400>  8

Asp Gly Asp Gly Tyr Ile Thr Thr Lys Glu
1               5                   10


<210>  9
<211>  10
<212>  PRT
<213>  artificial sequence

<220>
<223>  synthetic peptide EF-hand1 loop delta-K2D3/C7Y/T10A/K11A

<400>  9

Asp Gly Asp Gly Tyr Ile Thr Ala Ala Glu
1               5                   10


<210>  10
<211>  10
<212>  PRT
<213>  artificial sequence

<220>

<223>  synthetic peptide EF-hand2 loop delta-A2D3

<400>  10

Asp Gly Asn Gly Thr Ile Asp Phe Pro Glu
1               5                   10


<210>  11
<211>  10
<212>  PRT
<213>  artificial sequence

<220>
<223>  synthetic peptide EF-hand2 loop delta-A2D3/T7Y

<400>  11

Asp Gly Asn Gly Tyr Ile Asp Phe Pro Glu
1               5                   10


<210>  12
<211>  10
<212>  PRT
<213>  artificial sequence

<220>
<223>  synthetic peptide EF-hand2 loop delta-A2D3/N5D

<400>  12

Asp Gly Asp Gly Thr Ile Asp Phe Pro Glu
1               5                   10


<210>  13
<211>  10
<212>  PRT
<213>  artificial sequence

<220>
<223>  synthetic peptide EF-hand2 loop delta-A2D3/T7Y/N5D

<400>  13

Asp Gly Asp Gly Tyr Ile Asp Phe Pro Glu
1               5                   10


<210>  14
<211>  10
<212>  PRT
<213>  artificial sequence

<220>
<223>  synthetic peptide EF-hand3 loop delta-K2D3

<400>  14

Asp Gln Asn Gly Phe Ile Ser Ala Ala Glu
1               5                   10

<210>  15
<211>  10
<212>  PRT
<213>  artificial sequence

<220>
<223>  synthetic peptide EF-hand4 loop delta-V2D3

<400>  15

Asp Gly Asp Gly Gln Ile Asn Tyr Glu Glu
1               5                   10


<210>  16
<211>  228
<212>  DNA
<213>  artificial sequence

<220>
<223>  synthetic polynucleotide encoding CaM-delta

<400>  16
tccatggcgg atcagctcac cgacgatcag atctctgagt ttaaggaagc tttcagctta      60

ttcgacggtg atggttacat taccgccgcg gagctgggta ctgtgatgcg ttcccttgga     120

caaaacccaa ccgaagcaga gcttcaagac atgatcaacg aagtggctgc tgctggtaac     180

ggtaccattg atttcccaga gttcttgaac cttatggctc gtaagtga                  228


<210>  17
<211>  75
<212>  PRT
<213>  artificial sequence

<220>
<223>  synthetic peptide CaM-delta

<400>  17

Ser Met Ala Asp Gln Leu Thr Asp Asp Gln Ile Ser Glu Phe Lys Glu
1               5                   10                  15


Ala Phe Ser Leu Phe Asp Gly Asp Gly Tyr Ile Thr Ala Ala Glu Leu
            20                  25                  30


Gly Thr Val Met Arg Ser Leu Gly Gln Asn Pro Thr Glu Ala Glu Leu
        35                  40                  45


Gln Asp Met Ile Asn Glu Val Ala Ala Ala Gly Asn Gly Thr Ile Asp
    50                  55                  60


Phe Pro Glu Phe Leu Asn Leu Met Ala Arg Lys
65                  70                  75

<210>  18
<211>  147
<212>  PRT
<213>  artificial sequence

<220>
<223>  synthetic Calmodulin variant protein  from Cameleon
       biosensor-delta protein

<400>  18

Met Ala Asp Gln Leu Thr Asp Asp Gln Ile Ser Glu Phe Lys Glu Ala
1               5                   10                  15

Phe Ser Leu Phe Asp Gly Asp Gly Cys Ile Thr Thr Lys Glu Leu Gly
            20                  25                  30

Thr Val Met Arg Ser Leu Gly Gln Asn Pro Thr Glu Ala Glu Leu Gln
        35                  40                  45

Asp Met Ile Asn Glu Val Asp Ala Asp Gly Asn Gly Thr Ile Asp Phe
    50                  55                  60

Pro Glu Phe Leu Asn Leu Met Ala Arg Lys Met Lys Asp Thr Asp Ser
65                  70                  75                  80

Glu Glu Glu Leu Lys Glu Ala Phe Arg Val Phe Asp Lys Asp Gln Asn
                85                  90                  95

Gly Phe Ile Ser Ala Ala Glu Leu Arg His Val Met Thr Asn Leu Gly
            100                 105                 110

Glu Lys Leu Thr Asp Glu Glu Val Asp Glu Met Ile Lys Glu Ala Asp
        115                 120                 125

Val Asp Gly Asp Gly Gln Ile Asn Tyr Glu Glu Phe Val Lys Val Met
    130                 135                 140

Met Ala Lys
145


<210>  19
<211>  149
<212>  PRT
<213>  artificial sequence

<220>
<223>  synthetic Calmodulin variant protein from Cameleon biosensor
       -delta-S2M protein

<400>  19

Met Ala Asp Gln Leu Thr Asp Asp Gln Ile Ser Glu Phe Lys Glu Ala

1               5                   10                  15

Phe Ser Leu Phe Asp Lys Asp Gly Asp Gly Cys Ile Thr Thr Lys Glu
            20                  25                  30

Leu Gly Thr Val Met Arg Ser Leu Gly Gln Asn Pro Thr Glu Ala Glu
        35                  40                  45

Leu Gln Asp Met Ile Asn Glu Val Ala Ala Ala Gly Asn Gly Thr Ile
    50                  55                  60

Asp Phe Pro Glu Phe Leu Asn Leu Met Ala Arg Lys Met Lys Asp Thr
65                  70                  75                  80

Asp Ser Glu Glu Glu Leu Lys Glu Ala Phe Arg Val Phe Asp Lys Asp
                85                  90                  95

Gln Asn Gly Phe Ile Ser Ala Ala Glu Leu Arg His Val Met Thr Asn
            100                 105                 110

Leu Gly Glu Lys Leu Thr Asp Glu Glu Val Asp Glu Met Ile Lys Glu
        115                 120                 125

Ala Asp Val Asp Gly Asp Gly Gln Ile Asn Tyr Glu Glu Phe Val Lys
    130                 135                 140

Val Met Met Ala Lys
145

<210>  20
<211>  141
<212>  PRT
<213>  artificial sequence

<220>
<223>  synthetic calmodulin variant protein delta sites 1,2,3,4

<400>  20

Met Ala Asp Gln Leu Thr Asp Asp Gln Ile Ser Glu Phe Lys Glu Ala
1               5                   10                  15

Phe Ser Leu Phe Asp Gly Asp Gly Cys Ile Thr Thr Lys Glu Leu Gly
            20                  25                  30

Thr Val Met Arg Ser Leu Gly Gln Asn Pro Thr Glu Ala Glu Leu Gln
        35                  40                  45

Asp Met Ile Asn Glu Val Asp Gly Asn Gly Thr Ile Asp Phe Pro Glu
    50                  55                  60

Phe Leu Asn Leu Met Ala Arg Lys Met Lys Asp Thr Asp Ser Glu Glu
65                  70                  75                  80

Glu Leu Lys Glu Ala Phe Arg Val Phe Asp Gln Asn Gly Phe Ile Ser
                85                  90                  95

Ala Ala Glu Leu Arg His Val Met Thr Asn Leu Gly Glu Lys Leu Thr
            100                 105                 110

Asp Glu Glu Val Asp Glu Met Ile Lys Glu Ala Asp Gly Asp Gly Gln
        115                 120                 125

Ile Asn Tyr Glu Glu Phe Val Lys Val Met Met Ala Lys
    130                 135                 140

<210>  21
<211>  78
<212>  DNA
<213>  artificial sequence

<220>
<223>  synthetic polynucleotide encoding M13 peptide

<400>  21
aaacgtcgct ggaaaaaaaa ctttattgcg gtgagcgcgg ccaaccgctt taaaaaaatt        60

agctcgagcg gcgcgctg                                                    78

<210>  22
<211>  26
<212>  PRT
<213>  artificial sequence

<220>
<223>  synthetic peptide M13

<400>  22

Lys Arg Arg Trp Lys Lys Asn Phe Ile Ala Val Ser Ala Ala Asn Arg
1               5                   10                  15

Phe Lys Lys Ile Ser Ser Ser Gly Ala Leu
            20                  25

<210>  23
<211>  25
<212>  PRT
<213>  artificial sequence

<220>
<223>  synthetic peptide skMLCK

<400>  23

Lys Arg Arg Trp Lys Lys Asn Phe Ile Ala Val Ser Ala Ala Asn Arg
1               5                   10                  15

Phe Lys Lys Ile Ser Ser Ser Gly Ala
            20                  25

<210>  24
<211>  17
<212>  PRT
<213>  artificial sequence

<220>
<223>  synthetic peptide MLCKp

<400>  24

Arg Arg Lys Trp Gln Lys Thr Gly His Ala Val Arg Ala Ile Gly Arg
1               5                   10                  15

Leu

<210>  25
<211>  20
<212>  PRT
<213>  artificial sequence

<220>
<223>  synthetic peptide smMLCK

<400>  25

Ala Arg Arg Lys Trp Gln Lys Thr Gly His Ala Val Arg Ala Ile Gly
1               5                   10                  15

Arg Leu Ser Ser
            20

<210>  26
<211>  13
<212>  PRT
<213>  artificial sequence

<220>
<223>  synthetic peptide from wasp venom

<400>  26

Val Asn Trp Lys Lys Ile Gly Gln His Ile Leu Ser Val
1               5                   10

<210>  27
<211>  24
<212>  PRT
<213>  artificial sequence

<220>
<223>  synthetic peptide p21

<400>  27

Lys Arg Arg Gln Thr Ser Met Thr Asp Phe Tyr His Ser Lys Arg Arg
1               5                   10                  15

Leu Ile Phe Ser Lys Arg Lys Pro
            20

<210>  28
<211>  26
<212>  PRT
<213>  artificial sequence

<220>
<223>  synthetic peptide from melittin

<400>  28

Gln Gln Arg Lys Arg Lys Ile Trp Ser Ile Leu Ala Pro Leu Gly Thr
1               5                   10                  15

Thr Leu Val Lys Leu Val Ala Gly Ile Gly
            20                  25

<210>  29
<211>  24
<212>  PRT
<213>  artificial sequence

<220>
<223>  synthetic peptide from spectrin

<400>  29

Lys Thr Ala Ser Pro Trp Lys Ser Ala Arg Leu Met Val His Thr Val
1               5                   10                  15

Ala Thr Phe Asn Ser Ile Lys Glu
            20

<210>  30
<211>  22
<212>  PRT
<213>  artificial sequence

<220>
<223>  synthetic peptide CaMKI

<400>  30

Ala Lys Ser Lys Trp Lys Gln Ala Phe Asn Ala Thr Ala Val Val Arg
1               5                   10                  15

His Met Arg Lys Leu Gln
            20

<210>  31
<211>  25
<212>  PRT
<213>  artificial sequence

<220>
<223>  synthetic peptide CaMKII

<400>  31

Leu Lys Lys Phe Asn Ala Arg Arg Lys Leu Lys Gly Ala Ile Leu Thr
1               5                   10                  15

Thr Met Leu Ala Thr Arg Asn Phe Ser
            20                  25

<210>  32
<211>  27
<212>  PRT
<213>  artificial sequence

<220>
<223>  synthetic peptide CaMKK

<400>  32

Arg Phe Pro Asn Gly Phe Arg Lys Arg His Gly Met Ala Lys Val Leu
1               5                   10                  15

Ile Leu Thr Asp Leu Arg Pro Ile Arg Arg Val
            20                  25

<210>  33
<211>  17
<212>  PRT
<213>  artificial sequence

<220>
<223>  synthetic peptide1

<400>  33

Leu Lys Trp Lys Lys Leu Leu Lys Leu Leu Lys Lys Leu Leu Lys Leu
1               5                   10                  15

Gly

<210>  34
<211>  1953
<212>  DNA
<213>  artificial sequence

<220>
<223>  synthetic polynucleotide encoding biosensor-delta

<400>  34
tccatggtga gcaagggcga ggagctgttc accggggtgg tgcccatcct ggtcgagctg      60

gacggcgacg taaacggcca caggttcagc gtgtccggcg agggcgaggg cgatgccacc     120

tacggcaagc tgaccctgaa gttcatctgc accaccggca agctgcccgt gccctggccc     180

accctcgtga ccaccctgac ctggggcgtg cagtgcttca gccgctaccc cgaccacatg     240

aagcagcacg acttcttcaa gtccgccatg cccgaaggct acgtccagga gcgtaccatc     300

ttcttcaagg acgacggcaa ctacaagacc cgcgccgagg tgaagttcga gggcgacacc     360

ctggtgaacc gcatcgagct gaagggcatc gacttcaagg aggacggcaa catcctgggg     420

cacaagctgg agtacaacta catcagccac aacgtctata tcaccgccga caagcagaag     480

aacggcatca aggcccactt caagatccgc cacaacatcg aggacggcag cgtgcagctc     540

gccgaccact accagcagaa cacccccatc ggcgacggcc ccgtgctgct gcccgacaac     600

cactacctga gcacccagtc cgccctgagc aaagacccca acgagaagcg cgatcacatg     660

gtcctgctgg agttcgtgac cgccgccgag ctcatggcgg atcagttgac cgacgatcag     720

atctctgaat ttaaggaagc cttcagctta ttcgacggtg atggttgcat taccaccaag     780

gaactgggta ctgtgatgcg ttccctgggc caaaacccga ccgaagcaga gctgcaagac     840

atgatcaacg aagtggatgc ggatggtaac ggtaccattg atttcccgga attcttgaac     900

ctgatggccc gtaagatgaa agacaccgac agcgaggaag agctgaaaga agccttccgc     960

gttttcgaca aagaccagaa cggtttcatc agcgcagcgg aactgcgcca tgtgatgacc    1020

aacctgggcg aaaaactgac ggatgaagaa gttgatgaga tgatcaaaga agcggatgtg    1080

gatggtgatg gtcagattaa ctacgaagag tttgttaagg tgatgatggc gaaaggcggt    1140

ggcggtagca aacgtcgctg gaaaaaaaac tttattgcgg tgagcgcggc caaccgcttt    1200

aaaaaaatta gctcgagcgg cgcgctggtc gacatggtga gcaagggcga ggagctgttc    1260

accggggtgg tgcccatcct ggtcgagctg gacggcgacg taaacggcca caagttcagc    1320

gtgtccggcg agggcgaggg cgatgccacc tacggcaagc tgaccctgaa gttcatctgc    1380

accaccggca agctgcccgt gccctggccc accctcgtga ccaccttcgg ctacggcgtg    1440

cagtgcttcg cccgctaccc cgaccacatg aagcagcacg acttcttcaa gtccgccatg    1500

cccgaaggct acgtccagga gcgcaccatc ttcttcaagg acgacggcaa ctacaagacc    1560

cgcgccgagg tgaagttcga gggcgacacc ctggtgaacc gcatcgagct gaagggcatc    1620

gacttcaagg aggacggcaa catcctgggg cacaagctgg agtacaacta caacagccac    1680

aacgtctata tcatggccga caagcagaag aacggcatca aggtgaactt caagatccgc    1740

cacaacatcg aggacggcag cgtgcagctc gccgaccact accagcagaa cacccccatc     1800

ggcgacggcc ccgtgctgct gcccgacaac cactacctga gctaccagtc cgccctgagc     1860

aaagacccca acgagaagcg cgatcacatg gtcctgctgg agttcgtgac cgccgccggg     1920

atcactctcg gcatggacga gctgtacaag taa                                  1953

<210>  35
<211>  650
<212>  PRT
<213>  artificial sequence

<220>
<223>  synthetic protein biosensor-delta

<400>  35

Ser Met Val Ser Lys Gly Glu Glu Leu Phe Thr Gly Val Val Pro Ile
1               5                   10                  15

Leu Val Glu Leu Asp Gly Asp Val Asn Gly His Arg Phe Ser Val Ser
            20                  25                  30

Gly Glu Gly Glu Gly Asp Ala Thr Tyr Gly Lys Leu Thr Leu Lys Phe
        35                  40                  45

Ile Cys Thr Thr Gly Lys Leu Pro Val Pro Trp Pro Thr Leu Val Thr
    50                  55                  60

Thr Leu Thr Trp Gly Val Gln Cys Phe Ser Arg Tyr Pro Asp His Met
65                  70                  75                  80

Lys Gln His Asp Phe Phe Lys Ser Ala Met Pro Glu Gly Tyr Val Gln
                85                  90                  95

Glu Arg Thr Ile Phe Phe Lys Asp Asp Gly Asn Tyr Lys Thr Arg Ala
            100                 105                 110

Glu Val Lys Phe Glu Gly Asp Thr Leu Val Asn Arg Ile Glu Leu Lys
        115                 120                 125

Gly Ile Asp Phe Lys Glu Asp Gly Asn Ile Leu Gly His Lys Leu Glu
    130                 135                 140

Tyr Asn Tyr Ile Ser His Asn Val Tyr Ile Thr Ala Asp Lys Gln Lys
145                 150                 155                 160

Asn Gly Ile Lys Ala His Phe Lys Ile Arg His Asn Ile Glu Asp Gly
                165                 170                 175

Ser Val Gln Leu Ala Asp His Tyr Gln Gln Asn Thr Pro Ile Gly Asp
        180                 185                 190

Gly Pro Val Leu Leu Pro Asp Asn His Tyr Leu Ser Thr Gln Ser Ala
        195                 200                 205

Leu Ser Lys Asp Pro Asn Glu Lys Arg Asp His Met Val Leu Leu Glu
    210                 215                 220

Phe Val Thr Ala Ala Glu Leu Met Ala Asp Gln Leu Thr Asp Asp Gln
225                 230                 235                 240

Ile Ser Glu Phe Lys Glu Ala Phe Ser Leu Phe Asp Gly Asp Gly Cys
                245                 250                 255

Ile Thr Thr Lys Glu Leu Gly Thr Val Met Arg Ser Leu Gly Gln Asn
            260                 265                 270

Pro Thr Glu Ala Glu Leu Gln Asp Met Ile Asn Glu Val Asp Ala Asp
        275                 280                 285

Gly Asn Gly Thr Ile Asp Phe Pro Glu Phe Leu Asn Leu Met Ala Arg
    290                 295                 300

Lys Met Lys Asp Thr Asp Ser Glu Glu Glu Leu Lys Glu Ala Phe Arg
305                 310                 315                 320

Val Phe Asp Lys Asp Gln Asn Gly Phe Ile Ser Ala Ala Glu Leu Arg
                325                 330                 335

His Val Met Thr Asn Leu Gly Glu Lys Leu Thr Asp Glu Glu Val Asp
            340                 345                 350

Glu Met Ile Lys Glu Ala Asp Val Asp Gly Asp Gly Gln Ile Asn Tyr
        355                 360                 365

Glu Glu Phe Val Lys Val Met Met Ala Lys Gly Gly Gly Gly Ser Lys
    370                 375                 380

Arg Arg Trp Lys Lys Asn Phe Ile Ala Val Ser Ala Ala Asn Arg Phe
385                 390                 395                 400

Lys Lys Ile Ser Ser Ser Gly Ala Leu Val Asp Met Val Ser Lys Gly
                405                 410                 415

Glu Glu Leu Phe Thr Gly Val Val Pro Ile Leu Val Glu Leu Asp Gly
            420                 425                 430

Asp Val Asn Gly His Lys Phe Ser Val Ser Gly Glu Gly Glu Gly Asp
        435                 440                 445

Ala Thr Tyr Gly Lys Leu Thr Leu Lys Phe Ile Cys Thr Thr Gly Lys
    450                 455                 460

Leu Pro Val Pro Trp Pro Thr Leu Val Thr Thr Phe Gly Tyr Gly Val
465                 470                 475                 480

Gln Cys Phe Ala Arg Tyr Pro Asp His Met Lys Gln His Asp Phe Phe
                485                 490                 495

Lys Ser Ala Met Pro Glu Gly Tyr Val Gln Glu Arg Thr Ile Phe Phe
            500                 505                 510

Lys Asp Asp Gly Asn Tyr Lys Thr Arg Ala Glu Val Lys Phe Glu Gly
        515                 520                 525

Asp Thr Leu Val Asn Arg Ile Glu Leu Lys Gly Ile Asp Phe Lys Glu
    530                 535                 540

Asp Gly Asn Ile Leu Gly His Lys Leu Glu Tyr Asn Tyr Asn Ser His
545                 550                 555                 560

Asn Val Tyr Ile Met Ala Asp Lys Gln Lys Asn Gly Ile Lys Val Asn
                565                 570                 575

Phe Lys Ile Arg His Asn Ile Glu Asp Gly Ser Val Gln Leu Ala Asp
            580                 585                 590

His Tyr Gln Gln Asn Thr Pro Ile Gly Asp Gly Pro Val Leu Leu Pro
        595                 600                 605

Asp Asn His Tyr Leu Ser Tyr Gln Ser Ala Leu Ser Lys Asp Pro Asn
    610                 615                 620

Glu Lys Arg Asp His Met Val Leu Leu Glu Phe Val Thr Ala Ala Gly
625                 630                 635                 640

Ile Thr Leu Gly Met Asp Glu Leu Tyr Lys
                645                 650

<210>  36
<211>  1953
<212>  DNA
<213>  artificial sequence

<220>

<223>  synthetic polynucleotide encoding cameleon biosensor delta-S2M

<400>  36
tccatggtga gcaagggcga ggagctgttc accggggtgg tgcccatcct ggtcgagctg      60

gacggcgacg taaacggcca caggttcagc gtgtccggcg agggcgaggg cgatgccacc     120

tacggcaagc tgaccctgaa gttcatctgc accaccggca agctgcccgt gccctggccc     180

accctcgtga ccaccctgac ctggggcgtg cagtgcttca gccgctaccc cgaccacatg     240

aagcagcacg acttcttcaa gtccgccatg cccgaaggct acgtccagga gcgtaccatc     300

ttcttcaagg acgacggcaa ctacaagacc cgcgccgagg tgaagttcga gggcgacacc     360

ctggtgaacc gcatcgagct gaagggcatc gacttcaagg aggacggcaa catcctgggg     420

cacaagctgg agtacaacta catcagccac aacgtctata tcaccgccga caagcagaag     480

aacggcatca aggcccactt caagatccgc cacaacatcg aggacggcag cgtgcagctc     540

gccgaccact accagcagaa cacccccatc ggcgacggcc ccgtgctgct gcccgacaac     600

cactacctga gcacccagtc cgccctgagc aaagacccca acgagaagcg cgatcacatg     660

gtcctgctgg agttcgtgac cgccgccgag ctcatggcgg atcagttgac cgacgatcag     720

atctctgaat ttaaggaagc cttcagctta ttcgacggtg atggttgcat taccaccaag     780

gaactgggta ctgtgatgcg ttccctgggc caaaacccga ccgaagcaga gctgcaagac     840

atgatcaacg aagtggctgc ggctggtaac ggtaccattg atttcccgga attcttgaac     900

ctgatggccc gtaagatgaa agacaccgac agcgaggaag agctgaaaga agccttccgc     960

gttttcgaca aagaccagaa cggtttcatc agcgcagcgg aactgcgcca tgtgatgacc    1020

aacctgggcg aaaaactgac ggatgaagaa gttgatgaga tgatcaaaga agcggatgtg    1080

gatggtgatg gtcagattaa ctacgaagag tttgttaagg tgatgatggc gaaaggcggt    1140

ggcggtagca aacgtcgctg gaaaaaaaac tttattgcgg tgagcgcggc caaccgcttt    1200

aaaaaaatta gctcgagcgg cgcgctggtc gacatggtga gcaagggcga ggagctgttc    1260

accggggtgg tgcccatcct ggtcgagctg gacggcgacg taaacggcca caagttcagc    1320

gtgtccggcg agggcgaggg cgatgccacc tacggcaagc tgaccctgaa gttcatctgc    1380

accaccggca agctgcccgt gccctggccc accctcgtga ccaccttcgg ctacggcgtg    1440

cagtgcttcg cccgctaccc cgaccacatg aagcagcacg acttcttcaa gtccgccatg    1500

cccgaaggct acgtccagga gcgcaccatc ttcttcaagg acgacggcaa ctacaagacc    1560

cgcgccgagg tgaagttcga gggcgacacc ctggtgaacc gcatcgagct gaagggcatc    1620

gacttcaagg aggacggcaa catcctgggg cacaagctgg agtacaacta caacagccac    1680

aacgtctata tcatggccga caagcagaag aacggcatca aggtgaactt caagatccgc    1740

cacaacatcg aggacggcag cgtgcagctc gccgaccact accagcagaa cacccccatc    1800

ggcgacggcc ccgtgctgct gcccgacaac cactacctga gctaccagtc cgccctgagc 1860

aaagacccca acgagaagcg cgatcacatg gtcctgctgg agttcgtgac cgccgccggg 1920

atcactctcg gcatggacga gctgtacaag taa 1953

<210> 37
<211> 650
<212> PRT
<213> artificial sequence

<220>
<223> synthetic protein Cameleon biosensor delta-S2M

<400> 37

Ser Met Val Ser Lys Gly Glu Glu Leu Phe Thr Gly Val Val Pro Ile
1               5                   10                  15

Leu Val Glu Leu Asp Gly Asp Val Asn Gly His Arg Phe Ser Val Ser
            20                  25                  30

Gly Glu Gly Glu Gly Asp Ala Thr Tyr Gly Lys Leu Thr Leu Lys Phe
        35                  40                  45

Ile Cys Thr Thr Gly Lys Leu Pro Val Pro Trp Pro Thr Leu Val Thr
    50                  55                  60

Thr Leu Thr Trp Gly Val Gln Cys Phe Ser Arg Tyr Pro Asp His Met
65                  70                  75                  80

Lys Gln His Asp Phe Phe Lys Ser Ala Met Pro Glu Gly Tyr Val Gln
                85                  90                  95

Glu Arg Thr Ile Phe Phe Lys Asp Asp Gly Asn Tyr Lys Thr Arg Ala
            100                 105                 110

Glu Val Lys Phe Glu Gly Asp Thr Leu Val Asn Arg Ile Glu Leu Lys
        115                 120                 125

Gly Ile Asp Phe Lys Glu Asp Gly Asn Ile Leu Gly His Lys Leu Glu
    130                 135                 140

Tyr Asn Tyr Ile Ser His Asn Val Tyr Ile Thr Ala Asp Lys Gln Lys
145                 150                 155                 160

Asn Gly Ile Lys Ala His Phe Lys Ile Arg His Asn Ile Glu Asp Gly
                165                 170                 175

Ser Val Gln Leu Ala Asp His Tyr Gln Gln Asn Thr Pro Ile Gly Asp
            180                 185                 190

Gly Pro Val Leu Leu Pro Asp Asn His Tyr Leu Ser Thr Gln Ser Ala
        195                 200                 205

Leu Ser Lys Asp Pro Asn Glu Lys Arg Asp His Met Val Leu Leu Glu
    210                 215                 220

Phe Val Thr Ala Ala Glu Leu Met Ala Asp Gln Leu Thr Asp Asp Gln
225                 230                 235                 240

Ile Ser Glu Phe Lys Glu Ala Phe Ser Leu Phe Asp Gly Asp Gly Cys
                245                 250                 255

Ile Thr Thr Lys Glu Leu Gly Thr Val Met Arg Ser Leu Gly Gln Asn
            260                 265                 270

Pro Thr Glu Ala Glu Leu Gln Asp Met Ile Asn Glu Val Ala Ala Ala
        275                 280                 285

Gly Asn Gly Thr Ile Asp Phe Pro Glu Phe Leu Asn Leu Met Ala Arg
    290                 295                 300

Lys Met Lys Asp Thr Asp Ser Glu Glu Glu Leu Lys Glu Ala Phe Arg
305                 310                 315                 320

Val Phe Asp Lys Asp Gln Asn Gly Phe Ile Ser Ala Ala Glu Leu Arg
                325                 330                 335

His Val Met Thr Asn Leu Gly Glu Lys Leu Thr Asp Glu Glu Val Asp
            340                 345                 350

Glu Met Ile Lys Glu Ala Asp Val Asp Gly Asp Gly Gln Ile Asn Tyr
        355                 360                 365

Glu Glu Phe Val Lys Val Met Met Ala Lys Gly Gly Gly Gly Ser Lys
    370                 375                 380

Arg Arg Trp Lys Lys Asn Phe Ile Ala Val Ser Ala Ala Asn Arg Phe
385                 390                 395                 400

Lys Lys Ile Ser Ser Ser Gly Ala Leu Val Asp Met Val Ser Lys Gly
                405                 410                 415

Glu Glu Leu Phe Thr Gly Val Val Pro Ile Leu Val Glu Leu Asp Gly
            420                 425                 430

Asp Val Asn Gly His Lys Phe Ser Val Ser Gly Glu Gly Glu Gly Asp

435                 440                 445

Ala Thr Tyr Gly Lys Leu Thr Leu Lys Phe Ile Cys Thr Thr Gly Lys
    450                 455                 460

Leu Pro Val Pro Trp Pro Thr Leu Val Thr Thr Phe Gly Tyr Gly Val
465                 470                 475                 480

Gln Cys Phe Ala Arg Tyr Pro Asp His Met Lys Gln His Asp Phe Phe
                485                 490                 495

Lys Ser Ala Met Pro Glu Gly Tyr Val Gln Glu Arg Thr Ile Phe Phe
            500                 505                 510

Lys Asp Asp Gly Asn Tyr Lys Thr Arg Ala Glu Val Lys Phe Glu Gly
        515                 520                 525

Asp Thr Leu Val Asn Arg Ile Glu Leu Lys Gly Ile Asp Phe Lys Glu
    530                 535                 540

Asp Gly Asn Ile Leu Gly His Lys Leu Glu Tyr Asn Tyr Asn Ser His
545                 550                 555                 560

Asn Val Tyr Ile Met Ala Asp Lys Gln Lys Asn Gly Ile Lys Val Asn
                565                 570                 575

Phe Lys Ile Arg His Asn Ile Glu Asp Gly Ser Val Gln Leu Ala Asp
            580                 585                 590

His Tyr Gln Gln Asn Thr Pro Ile Gly Asp Gly Pro Val Leu Leu Pro
        595                 600                 605

Asp Asn His Tyr Leu Ser Tyr Gln Ser Ala Leu Ser Lys Asp Pro Asn
    610                 615                 620

Glu Lys Arg Asp His Met Val Leu Leu Glu Phe Val Thr Ala Ala Gly
625                 630                 635                 640

Ile Thr Leu Gly Met Asp Glu Leu Tyr Lys
                645                 650

<210>  38
<211>  644
<212>  PRT
<213>  artificial sequence

<220>
<223>  synthetic protein Cameleon biosensor delta-delta-delta-delta

<400>  38

Ser Met Val Ser Lys Gly Glu Glu Leu Phe Thr Gly Val Val Pro Ile
1               5                   10                  15

Leu Val Glu Leu Asp Gly Asp Val Asn Gly His Arg Phe Ser Val Ser
            20                  25                  30

Gly Glu Gly Glu Gly Asp Ala Thr Tyr Gly Lys Leu Thr Leu Lys Phe
        35                  40                  45

Ile Cys Thr Thr Gly Lys Leu Pro Val Pro Trp Pro Thr Leu Val Thr
    50                  55                  60

Thr Leu Thr Trp Gly Val Gln Cys Phe Ser Arg Tyr Pro Asp His Met
65                  70                  75                  80

Lys Gln His Asp Phe Phe Lys Ser Ala Met Pro Glu Gly Tyr Val Gln
                85                  90                  95

Glu Arg Thr Ile Phe Phe Lys Asp Asp Gly Asn Tyr Lys Thr Arg Ala
            100                 105                 110

Glu Val Lys Phe Glu Gly Asp Thr Leu Val Asn Arg Ile Glu Leu Lys
        115                 120                 125

Gly Ile Asp Phe Lys Glu Asp Gly Asn Ile Leu Gly His Lys Leu Glu
    130                 135                 140

Tyr Asn Tyr Ile Ser His Asn Val Tyr Ile Thr Ala Asp Lys Gln Lys
145                 150                 155                 160

Asn Gly Ile Lys Ala His Phe Lys Ile Arg His Asn Ile Glu Asp Gly
                165                 170                 175

Ser Val Gln Leu Ala Asp His Tyr Gln Gln Asn Thr Pro Ile Gly Asp
            180                 185                 190

Gly Pro Val Leu Leu Pro Asp Asn His Tyr Leu Ser Thr Gln Ser Ala
        195                 200                 205

Leu Ser Lys Asp Pro Asn Glu Lys Arg Asp His Met Val Leu Leu Glu
    210                 215                 220

Phe Val Thr Ala Ala Glu Leu Met Ala Asp Gln Leu Thr Asp Asp Gln
225                 230                 235                 240

Ile Ser Glu Phe Lys Glu Ala Phe Ser Leu Phe Asp Gly Asp Gly Cys

245                 250                 255

Ile Thr Thr Lys Glu Leu Gly Thr Val Met Arg Ser Leu Gly Gln Asn
            260                 265                 270

Pro Thr Glu Ala Glu Leu Gln Asp Met Ile Asn Glu Val Asp Gly Asn
        275                 280                 285

Gly Thr Ile Asp Phe Pro Glu Phe Leu Asn Leu Met Ala Arg Lys Met
    290                 295                 300

Lys Asp Thr Asp Ser Glu Glu Glu Leu Lys Glu Ala Phe Arg Val Phe
305                 310                 315                 320

Asp Gln Asn Gly Phe Ile Ser Ala Ala Glu Leu Arg His Val Met Thr
                325                 330                 335

Asn Leu Gly Glu Lys Leu Thr Asp Glu Glu Val Asp Glu Met Ile Lys
            340                 345                 350

Glu Ala Asp Gly Asp Gly Gln Ile Asn Tyr Glu Glu Phe Val Lys Val
        355                 360                 365

Met Met Ala Lys Gly Gly Gly Gly Ser Lys Arg Arg Trp Lys Lys Asn
    370                 375                 380

Phe Ile Ala Val Ser Ala Ala Asn Arg Phe Lys Lys Ile Ser Ser Ser
385                 390                 395                 400

Gly Ala Leu Val Asp Met Val Ser Lys Gly Glu Glu Leu Phe Thr Gly
                405                 410                 415

Val Val Pro Ile Leu Val Glu Leu Asp Gly Asp Val Asn Gly His Lys
            420                 425                 430

Phe Ser Val Ser Gly Glu Gly Glu Gly Asp Ala Thr Tyr Gly Lys Leu
        435                 440                 445

Thr Leu Lys Phe Ile Cys Thr Thr Gly Lys Leu Pro Val Pro Trp Pro
    450                 455                 460

Thr Leu Val Thr Thr Phe Gly Tyr Gly Val Gln Cys Phe Ala Arg Tyr
465                 470                 475                 480

Pro Asp His Met Lys Gln His Asp Phe Phe Lys Ser Ala Met Pro Glu
                485                 490                 495

Gly Tyr Val Gln Glu Arg Thr Ile Phe Phe Lys Asp Asp Gly Asn Tyr
            500                 505                 510

Lys Thr Arg Ala Glu Val Lys Phe Glu Gly Asp Thr Leu Val Asn Arg
        515                 520                 525

Ile Glu Leu Lys Gly Ile Asp Phe Lys Glu Asp Gly Asn Ile Leu Gly
    530                 535                 540

His Lys Leu Glu Tyr Asn Tyr Asn Ser His Asn Val Tyr Ile Met Ala
545                 550                 555                 560

Asp Lys Gln Lys Asn Gly Ile Lys Val Asn Phe Lys Ile Arg His Asn
                565                 570                 575

Ile Glu Asp Gly Ser Val Gln Leu Ala Asp His Tyr Gln Gln Asn Thr
            580                 585                 590

Pro Ile Gly Asp Gly Pro Val Leu Leu Pro Asp Asn His Tyr Leu Ser
        595                 600                 605

Tyr Gln Ser Ala Leu Ser Lys Asp Pro Asn Glu Lys Arg Asp His Met
    610                 615                 620

Val Leu Leu Glu Phe Val Thr Ala Ala Gly Ile Thr Leu Gly Met Asp
625                 630                 635                 640

Glu Leu Tyr Lys

<210>  39
<211>  234
<212>  DNA
<213>  artificial sequence

<220>
<223>  synthetic polynucleotide encoding CaM1

<400>  39
tccatggcgg atcagctcac cgacgatcag atctctgagt ttaaggaagc tttcagctta      60

ttcgacaagg atggtgatgg ttacattacc gccgcggagc tgggtactgt gatgcgttcc     120

cttggacaaa acccaaccga agcagagctt caagacatga tcaacgaagt ggctgctgct     180

ggtaacggta ccattgattt cccagagttc ttgaacctta tggctcgtaa gtga           234

<210>  40
<211>  77
<212>  PRT
<213>  artificial sequence

<220>
<223>  synthetic peptide CaM1

<400>  40

Ser Met Ala Asp Gln Leu Thr Asp Asp Gln Ile Ser Glu Phe Lys Glu
1               5                   10                  15

Ala Phe Ser Leu Phe Asp Lys Asp Gly Asp Gly Tyr Ile Thr Ala Ala
            20                  25                  30

Glu Leu Gly Thr Val Met Arg Ser Leu Gly Gln Asn Pro Thr Glu Ala
        35                  40                  45

Glu Leu Gln Asp Met Ile Asn Glu Val Ala Ala Ala Gly Asn Gly Thr
    50                  55                  60

Ile Asp Phe Pro Glu Phe Leu Asn Leu Met Ala Arg Lys
65                  70                  75

<210>  41
<211>  45
<212>  DNA
<213>  artificial sequence

<220>
<223>  synthetic oligonucleotide primer DGD S

<400>  41
gaagctttca gcttattcga cggtgatggt tacattaccg ccgcg                        45

<210>  42
<211>  45
<212>  DNA
<213>  artificial sequence

<220>
<223>  synthetic oligonucleotide primer DGD AS

<400>  42
cgcggcggta atgtaaccat caccgtcgaa taagctgaaa gcttc                        45

<210>  43
<211>  52
<212>  DNA
<213>  artificial sequence

<220>
<223>  synthetic oligonucleotide primer S-TEV-eCFP- BamHI

<400>  43
gagaggatcc gagaacctgt acttccagtc catggtgagc aagggcgagg ag                52

<210>  44
<211>  33

<212>  DNA
<213>  artificial sequence

<220>
<223>  synthetic oligonucleotide primer AS-eCFP-SacI

<400>  44
taaagagctc ggcggcggtc acgaactcca gca                                    33


<210>  45
<211>  31
<212>  DNA
<213>  artificial sequence

<220>
<223>  synthetic oligonucleotide primer S-eYFP- SalI

<400>  45
tatagtcgac atggtgagca agggcgagga g                                      31


<210>  46
<211>  35
<212>  DNA
<213>  artificial sequence

<220>
<223>  synthetic oligonucleotide primer AS-eYFP-HindIII

<400>  46
gggcaagctt ttacttgtac agctcgtcca tgccg                                  35


<210>  47
<211>  1959
<212>  DNA
<213>  artificial sequence

<220>
<223>  synthetic polynucleotide encoding Cameleon biosensor WT

<400>  47
tccatggtga gcaagggcga ggagctgttc accggggtgg tgcccatcct ggtcgagctg      60

gacggcgacg taaacggcca caggttcagc gtgtccggcg agggcgaggg cgatgccacc     120

tacggcaagc tgaccctgaa gttcatctgc accaccggca agctgcccgt gccctggccc     180

accctcgtga ccaccctgac ctggggcgtg cagtgcttca gccgctaccc cgaccacatg     240

aagcagcacg acttcttcaa gtccgccatg cccgaaggct acgtccagga gcgtaccatc     300

ttcttcaagg acgacggcaa ctacaagacc cgcgccgagg tgaagttcga gggcgacacc     360

ctggtgaacc gcatcgagct gaagggcatc gacttcaagg aggacggcaa catcctgggg     420

cacaagctgg agtacaacta catcagccac aacgtctata tcaccgccga caagcagaag     480

aacggcatca aggcccactt caagatccgc cacaacatcg aggacggcag cgtgcagctc     540

gccgaccact accagcagaa cacccccatc ggcgacggcc ccgtgctgct gcccgacaac     600

cactacctga gcacccagtc cgccctgagc aaagacccca acgagaagcg cgatcacatg 660

gtcctgctgg agttcgtgac cgccgccgag ctcatggcgg atcagttgac cgacgatcag 720

atctctgaat ttaaggaagc cttcagctta ttcgacaaag atggtgatgg ttgcattacc 780

accaaggaac tgggtactgt gatgcgttcc ctgggccaaa acccgaccga agcagagctg 840

caagacatga tcaacgaagt ggatgcggat ggtaacggta ccattgattt cccggaattc 900

ttgaacctga tggcccgtaa gatgaaagac accgacagcg aggaagagct gaaagaagcc 960

ttccgcgttt tcgacaaaga ccagaacggt ttcatcagcg cagcggaact gcgccatgtg 1020

atgaccaacc tgggcgaaaa actgacggat gaagaagttg atgagatgat caaagaagcg 1080

gatgtggatg gtgatggtca gattaactac gaagagtttg ttaaggtgat gatggcgaaa 1140

ggcggtggcg gtagcaaacg tcgctggaaa aaaaacttta ttgcggtgag cgcggccaac 1200

cgctttaaaa aaattagctc gagcggcgcg ctggtcgaca tggtgagcaa gggcgaggag 1260

ctgttcaccg gggtggtgcc catcctggtc gagctggacg gcgacgtaaa cggccacaag 1320

ttcagcgtgt ccggcgaggg cgagggcgat gccacctacg gcaagctgac cctgaagttc 1380

atctgcacca ccggcaagct gcccgtgccc tggcccaccc tcgtgaccac cttcggctac 1440

ggcgtgcagt gcttcgcccg ctaccccgac cacatgaagc agcacgactt cttcaagtcc 1500

gccatgcccg aaggctacgt ccaggagcgc accatcttct tcaaggacga cggcaactac 1560

aagacccgcg ccgaggtgaa gttcgagggc gacaccctgg tgaaccgcat cgagctgaag 1620

ggcatcgact tcaaggagga cggcaacatc ctggggcaca agctggagta caactacaac 1680

agccacaacg tctatatcat ggccgacaag cagaagaacg gcatcaaggt gaacttcaag 1740

atccgccaca acatcgagga cggcagcgtg cagctcgccg accactacca gcagaacacc 1800

cccatcggcg acggccccgt gctgctgccc gacaaccact acctgagcta ccagtccgcc 1860

ctgagcaaag accccaacga gaagcgcgat cacatggtcc tgctggagtt cgtgaccgcc 1920

gccgggatca ctctcggcat ggacgagctg tacaagtaa 1959

<210> 48
<211> 652
<212> PRT
<213> artificial sequence

<220>
<223> synthetic protein Cameleon biosensor WT

<400> 48

Ser Met Val Ser Lys Gly Glu Glu Leu Phe Thr Gly Val Val Pro Ile
1               5                   10                  15

Leu Val Glu Leu Asp Gly Asp Val Asn Gly His Arg Phe Ser Val Ser
            20                  25                  30

Gly Glu Gly Glu Gly Asp Ala Thr Tyr Gly Lys Leu Thr Leu Lys Phe
        35                  40                  45

Ile Cys Thr Thr Gly Lys Leu Pro Val Pro Trp Pro Thr Leu Val Thr
    50                  55                  60

Thr Leu Thr Trp Gly Val Gln Cys Phe Ser Arg Tyr Pro Asp His Met
65                  70                  75                  80

Lys Gln His Asp Phe Phe Lys Ser Ala Met Pro Glu Gly Tyr Val Gln
                85                  90                  95

Glu Arg Thr Ile Phe Phe Lys Asp Asp Gly Asn Tyr Lys Thr Arg Ala
            100                 105                 110

Glu Val Lys Phe Glu Gly Asp Thr Leu Val Asn Arg Ile Glu Leu Lys
        115                 120                 125

Gly Ile Asp Phe Lys Glu Asp Gly Asn Ile Leu Gly His Lys Leu Glu
    130                 135                 140

Tyr Asn Tyr Ile Ser His Asn Val Tyr Ile Thr Ala Asp Lys Gln Lys
145                 150                 155                 160

Asn Gly Ile Lys Ala His Phe Lys Ile Arg His Asn Ile Glu Asp Gly
                165                 170                 175

Ser Val Gln Leu Ala Asp His Tyr Gln Gln Asn Thr Pro Ile Gly Asp
            180                 185                 190

Gly Pro Val Leu Leu Pro Asp Asn His Tyr Leu Ser Thr Gln Ser Ala
        195                 200                 205

Leu Ser Lys Asp Pro Asn Glu Lys Arg Asp His Met Val Leu Leu Glu
    210                 215                 220

Phe Val Thr Ala Ala Glu Leu Met Ala Asp Gln Leu Thr Asp Asp Gln
225                 230                 235                 240

Ile Ser Glu Phe Lys Glu Ala Phe Ser Leu Phe Asp Lys Asp Gly Asp
                245                 250                 255

Gly Cys Ile Thr Thr Lys Glu Leu Gly Thr Val Met Arg Ser Leu Gly
            260                 265                 270

Gln Asn Pro Thr Glu Ala Glu Leu Gln Asp Met Ile Asn Glu Val Asp

275                 280                 285

Ala Asp Gly Asn Gly Thr Ile Asp Phe Pro Glu Phe Leu Asn Leu Met
    290                 295                 300

Ala Arg Lys Met Lys Asp Thr Asp Ser Glu Glu Glu Leu Lys Glu Ala
305                 310                 315                 320

Phe Arg Val Phe Asp Lys Asp Gln Asn Gly Phe Ile Ser Ala Ala Glu
                325                 330                 335

Leu Arg His Val Met Thr Asn Leu Gly Glu Lys Leu Thr Asp Glu Glu
            340                 345                 350

Val Asp Glu Met Ile Lys Glu Ala Asp Val Asp Gly Asp Gly Gln Ile
        355                 360                 365

Asn Tyr Glu Glu Phe Val Lys Val Met Met Ala Lys Gly Gly Gly Gly
    370                 375                 380

Ser Lys Arg Arg Trp Lys Lys Asn Phe Ile Ala Val Ser Ala Ala Asn
385                 390                 395                 400

Arg Phe Lys Lys Ile Ser Ser Ser Gly Ala Leu Val Asp Met Val Ser
                405                 410                 415

Lys Gly Glu Glu Leu Phe Thr Gly Val Val Pro Ile Leu Val Glu Leu
            420                 425                 430

Asp Gly Asp Val Asn Gly His Lys Phe Ser Val Ser Gly Glu Gly Glu
        435                 440                 445

Gly Asp Ala Thr Tyr Gly Lys Leu Thr Leu Lys Phe Ile Cys Thr Thr
    450                 455                 460

Gly Lys Leu Pro Val Pro Trp Pro Thr Leu Val Thr Thr Phe Gly Tyr
465                 470                 475                 480

Gly Val Gln Cys Phe Ala Arg Tyr Pro Asp His Met Lys Gln His Asp
                485                 490                 495

Phe Phe Lys Ser Ala Met Pro Glu Gly Tyr Val Gln Glu Arg Thr Ile
            500                 505                 510

Phe Phe Lys Asp Asp Gly Asn Tyr Lys Thr Arg Ala Glu Val Lys Phe
        515                 520                 525

Glu Gly Asp Thr Leu Val Asn Arg Ile Glu Leu Lys Gly Ile Asp Phe
    530                 535                 540

Lys Glu Asp Gly Asn Ile Leu Gly His Lys Leu Glu Tyr Asn Tyr Asn
545                 550                 555                 560

Ser His Asn Val Tyr Ile Met Ala Asp Lys Gln Lys Asn Gly Ile Lys
                565                 570                 575

Val Asn Phe Lys Ile Arg His Asn Ile Glu Asp Gly Ser Val Gln Leu
            580                 585                 590

Ala Asp His Tyr Gln Gln Asn Thr Pro Ile Gly Asp Gly Pro Val Leu
        595                 600                 605

Leu Pro Asp Asn His Tyr Leu Ser Tyr Gln Ser Ala Leu Ser Lys Asp
    610                 615                 620

Pro Asn Glu Lys Arg Asp His Met Val Leu Leu Glu Phe Val Thr Ala
625                 630                 635                 640

Ala Gly Ile Thr Leu Gly Met Asp Glu Leu Tyr Lys
                645                 650

<210>  49
<211>  40
<212>  DNA
<213>  artificial sequence

<220>
<223>  synthetic oligonucleotide primer  S-delta

<400>  49
ggaagccttc agcttattcg acggtgatgg ttgcattacc                              40

<210>  50
<211>  40
<212>  DNA
<213>  artificial sequence

<220>
<223>  synthetic oligonucleotide primer AS-delta

<400>  50
ggtaatgcaa ccatcaccgt cgaataagct gaaggcttcc                              40

<210>  51
<211>  34
<212>  DNA
<213>  artificial sequence

<220>
<223>  synthetic oligonucleotide primer S-S2M

<400>  51
ggtaccgtta ccagcagcag ccacttcgtt gatc                                    34


<210>  52
<211>  34
<212>  DNA
<213>  artificial sequence

<220>
<223>  synthetic oligonucleotide primer AS-S2M

<400>  52
gatcaacgaa gtggctgctg ctggtaacgg tacc                                    34


<210>  53
<211>  1959
<212>  DNA
<213>  artificial sequence

<220>
<223>  synthetic polynucleotide encoding Cameleon biosensor WT-S2M

<400>  53
tccatggtga gcaagggcga ggagctgttc accggggtgg tgcccatcct ggtcgagctg        60

gacggcgacg taaacggcca caggttcagc gtgtccggcg agggcgaggg cgatgccacc       120

tacggcaagc tgaccctgaa gttcatctgc accaccggca agctgcccgt gccctggccc       180

accctcgtga ccaccctgac ctggggcgtg cagtgcttca gccgctaccc cgaccacatg       240

aagcagcacg acttcttcaa gtccgccatg cccgaaggct acgtccagga gcgtaccatc       300

ttcttcaagg acgacggcaa ctacaagacc cgcgccgagg tgaagttcga gggcgacacc       360

ctggtgaacc gcatcgagct gaagggcatc gacttcaagg aggacggcaa catcctgggg       420

cacaagctgg agtacaacta catcagccac aacgtctata tcaccgccga caagcagaag       480

aacggcatca aggcccactt caagatccgc cacaacatcg aggacggcag cgtgcagctc       540

gccgaccact accagcagaa cacccccatc ggcgacggcc ccgtgctgct gcccgacaac       600

cactacctga gcacccagtc cgccctgagc aaagacccca acgagaagcg cgatcacatg       660

gtcctgctgg agttcgtgac cgccgccgag ctcatggcgg atcagttgac cgacgatcag       720

atctctgaat ttaaggaagc cttcagctta ttcgacaaag atggtgatgg ttgcattacc       780

accaaggaac tgggtactgt gatgcgttcc ctgggccaaa acccgaccga agcagagctg       840

caagacatga tcaacgaagt ggctgcggct ggtaacggta ccattgattt cccggaattc       900

ttgaacctga tggcccgtaa gatgaaagac accgacagcg aggaagagct gaaagaagcc       960

ttccgcgttt tcgacaaaga ccagaacggt ttcatcagcg cagcggaact gcgccatgtg      1020

atgaccaacc tgggcgaaaa actgacggat gaagaagttg atgagatgat caaagaagcg      1080

gatgtggatg gtgatggtca gattaactac gaagagtttg ttaaggtgat gatggcgaaa      1140

ggcggtggcg gtagcaaacg tcgctggaaa aaaaacttta ttgcggtgag cgcggccaac 1200

cgctttaaaa aaattagctc gagcggcgcg ctggtcgaca tggtgagcaa gggcgaggag 1260

ctgttcaccg gggtggtgcc catcctggtc gagctggacg gcgacgtaaa cggccacaag 1320

ttcagcgtgt ccggcgaggg cgagggcgat gccacctacg gcaagctgac cctgaagttc 1380

atctgcacca ccggcaagct gcccgtgccc tggcccaccc tcgtgaccac cttcggctac 1440

ggcgtgcagt gcttcgcccg ctaccccgac cacatgaagc agcacgactt cttcaagtcc 1500

gccatgcccg aaggctacgt ccaggagcgc accatcttct tcaaggacga cggcaactac 1560

aagacccgcg ccgaggtgaa gttcgagggc gacaccctgg tgaaccgcat cgagctgaag 1620

ggcatcgact tcaaggagga cggcaacatc ctggggcaca agctggagta caactacaac 1680

agccacaacg tctatatcat ggccgacaag cagaagaacg gcatcaaggt gaacttcaag 1740

atccgccaca acatcgagga cggcagcgtg cagctcgccg accactacca gcagaacacc 1800

cccatcggcg acggccccgt gctgctgccc gacaaccact acctgagcta ccagtccgcc 1860

ctgagcaaag accccaacga gaagcgcgat cacatggtcc tgctggagtt cgtgaccgcc 1920

gccgggatca ctctcggcat ggacgagctg tacaagtaa 1959

<210> 54
<211> 652
<212> PRT
<213> artificial sequence

<220>
<223> synthetic protein Cameleon biosensor WT-S2M

<400> 54

Ser Met Val Ser Lys Gly Glu Glu Leu Phe Thr Gly Val Val Pro Ile
1               5                   10                  15

Leu Val Glu Leu Asp Gly Asp Val Asn Gly His Arg Phe Ser Val Ser
            20                  25                  30

Gly Glu Gly Glu Gly Asp Ala Thr Tyr Gly Lys Leu Thr Leu Lys Phe
        35                  40                  45

Ile Cys Thr Thr Gly Lys Leu Pro Val Pro Trp Pro Thr Leu Val Thr
    50                  55                  60

Thr Leu Thr Trp Gly Val Gln Cys Phe Ser Arg Tyr Pro Asp His Met
65                  70                  75                  80

Lys Gln His Asp Phe Phe Lys Ser Ala Met Pro Glu Gly Tyr Val Gln
                85                  90                  95

Glu Arg Thr Ile Phe Phe Lys Asp Asp Gly Asn Tyr Lys Thr Arg Ala
            100                 105                 110

Glu Val Lys Phe Glu Gly Asp Thr Leu Val Asn Arg Ile Glu Leu Lys
        115                 120                 125

Gly Ile Asp Phe Lys Glu Asp Gly Asn Ile Leu Gly His Lys Leu Glu
    130                 135                 140

Tyr Asn Tyr Ile Ser His Asn Val Tyr Ile Thr Ala Asp Lys Gln Lys
145                 150                 155                 160

Asn Gly Ile Lys Ala His Phe Lys Ile Arg His Asn Ile Glu Asp Gly
                165                 170                 175

Ser Val Gln Leu Ala Asp His Tyr Gln Gln Asn Thr Pro Ile Gly Asp
            180                 185                 190

Gly Pro Val Leu Leu Pro Asp Asn His Tyr Leu Ser Thr Gln Ser Ala
        195                 200                 205

Leu Ser Lys Asp Pro Asn Glu Lys Arg Asp His Met Val Leu Leu Glu
    210                 215                 220

Phe Val Thr Ala Ala Glu Leu Met Ala Asp Gln Leu Thr Asp Asp Gln
225                 230                 235                 240

Ile Ser Glu Phe Lys Glu Ala Phe Ser Leu Phe Asp Lys Asp Gly Asp
                245                 250                 255

Gly Cys Ile Thr Thr Lys Glu Leu Gly Thr Val Met Arg Ser Leu Gly
            260                 265                 270

Gln Asn Pro Thr Glu Ala Glu Leu Gln Asp Met Ile Asn Glu Val Ala
        275                 280                 285

Ala Ala Gly Asn Gly Thr Ile Asp Phe Pro Glu Phe Leu Asn Leu Met
    290                 295                 300

Ala Arg Lys Met Lys Asp Thr Asp Ser Glu Glu Glu Leu Lys Glu Ala
305                 310                 315                 320

Phe Arg Val Phe Asp Lys Asp Gln Asn Gly Phe Ile Ser Ala Ala Glu
                325                 330                 335

Leu Arg His Val Met Thr Asn Leu Gly Glu Lys Leu Thr Asp Glu Glu
            340                 345                 350

Val Asp Glu Met Ile Lys Glu Ala Asp Val Asp Gly Asp Gly Gln Ile
        355                 360                 365

Asn Tyr Glu Glu Phe Val Lys Val Met Met Ala Lys Gly Gly Gly Gly
    370                 375                 380

Ser Lys Arg Arg Trp Lys Lys Asn Phe Ile Ala Val Ser Ala Ala Asn
385                 390                 395                 400

Arg Phe Lys Lys Ile Ser Ser Ser Gly Ala Leu Val Asp Met Val Ser
                405                 410                 415

Lys Gly Glu Glu Leu Phe Thr Gly Val Val Pro Ile Leu Val Glu Leu
            420                 425                 430

Asp Gly Asp Val Asn Gly His Lys Phe Ser Val Ser Gly Glu Gly Glu
        435                 440                 445

Gly Asp Ala Thr Tyr Gly Lys Leu Thr Leu Lys Phe Ile Cys Thr Thr
    450                 455                 460

Gly Lys Leu Pro Val Pro Trp Pro Thr Leu Val Thr Thr Phe Gly Tyr
465                 470                 475                 480

Gly Val Gln Cys Phe Ala Arg Tyr Pro Asp His Met Lys Gln His Asp
                485                 490                 495

Phe Phe Lys Ser Ala Met Pro Glu Gly Tyr Val Gln Glu Arg Thr Ile
            500                 505                 510

Phe Phe Lys Asp Asp Gly Asn Tyr Lys Thr Arg Ala Glu Val Lys Phe
        515                 520                 525

Glu Gly Asp Thr Leu Val Asn Arg Ile Glu Leu Lys Gly Ile Asp Phe
    530                 535                 540

Lys Glu Asp Gly Asn Ile Leu Gly His Lys Leu Glu Tyr Asn Tyr Asn
545                 550                 555                 560

Ser His Asn Val Tyr Ile Met Ala Asp Lys Gln Lys Asn Gly Ile Lys
                565                 570                 575

Val Asn Phe Lys Ile Arg His Asn Ile Glu Asp Gly Ser Val Gln Leu
            580                 585                 590

Ala Asp His Tyr Gln Gln Asn Thr Pro Ile Gly Asp Gly Pro Val Leu

        595                 600                 605

Leu Pro Asp Asn His Tyr Leu Ser Tyr Gln Ser Ala Leu Ser Lys Asp
    610                 615                 620

Pro Asn Glu Lys Arg Asp His Met Val Leu Leu Glu Phe Val Thr Ala
625                 630                 635                 640

Ala Gly Ile Thr Leu Gly Met Asp Glu Leu Tyr Lys
                645                 650

## Description

The present relates to new uranium-chelating peptides derived from EF-hand calcium-binding motif, useful for uranium biodetection and biodecontamination.

Uranium is a radioactive heavy metal, which is naturally present in varying concentrations in the environment. However, the wide use of uranium for industrial and military applications increases the risk of its distribution in the environment, which is aggravated by such factors as mining activities, uranium processing, or leaching of radioactive wastes.

Uranium presents radiological and chemical toxicity to living organisms. The linear dioxo uranyl form (UO₂²⁺) which corresponds to uranium in its hexavalent oxidation state U(VI) is the prevalent form of uranium in the presence of oxygen and the most stable chemical form of uranium in water. It is soluble, bioavailable, and thus potentially toxic.

Therefore, it is of high interest to develop systems allowing uranyl detection in the environment as well as molecules allowing its complexation in various environments with high affinity and selectivity. In particular, sensitive and easy to use systems allowing the detection of uranyl content in waters at the concentration levels defined by the international radioprotection authorities (inferior to 0.03 mg/L, *i.e.* 126 nM) would be of great help for the water level monitoring.

Among these systems, molecules derived from peptides are advantageous in that they are non toxic, and can be easily produced either synthetically or using recombinant techniques. Similarly, the development of organisms bearing uranyl biosensors, capable of detecting the uranyl fraction that is bioavailable and thus potentially toxic, is needed to assess water quality on a long term basis.

Finally, bioremediation is an emerging technique that would allow the decontamination of large areas of contaminated water and/or soils.

The helix-loop-helix calcium binding motif (EF-hand motif) is the most prevalent Ca²⁺ binding site in proteins. The canonical EF-hand motif of about 30 amino acids in length is structured by two alpha-helices bridged by a flexible metal-binding loop (also referred to as calcium-binding loop, calcium-binding site, loop or site) composed of 12 highly conserved residues, with calcium coordinating positions at 1 (+X), 3 (+Y), 5 (+Z), 7 (-Y), 9 (-X) and 12 (-Z). The residues at the ligand positions 1, 3, 5 and 12 and also at the non-ligand positions 6 and 8 are highly conserved. The calcium ion is coordinated in a pentagonal bipyramidal configuration with a coordination number of 7 to side-chain oxygen bearing groups at positions 1, 3, 5 and 12, a water molecule hydrogen bonded to residue at position 9, and the main chain carbonyl group of residue 7. The pentagonal bipyramidal configuration arises from two additional ligands provided by a bidendate glutamic or a monodentate aspartic acid which stabilizes a water ligand, in position 12. The residue at position 1 of the loop is most frequently occupied by an aspartate (D); two residues are found at position 3: aspartic acid (D) or asparagine (N), but most frequently D; position 5 is most often occupied by aspartic acid (D), serine (S) or asparagine (N); the residue at position 7 is variable; position 9 shows a preference for residues with a side-chain oxygen (D, N, E, S, T), and the residues more frequently found at position 12 are glutamate (E) and aspartate (D), most frequently E. The highly conserved residues at the non ligand positions 6 and 8 are Gly (G) and Ile (I), respectively. The majority of the known EF-hand Calcium-Binding proteins (CaBPs) contain paired EF-hand motifs.

Functionally, the EF-hand proteins can be divided into two classes: 1) signaling proteins and 2) buffering/transport proteins. The first group is the largest and includes the most well-known members of the family such as calmodulin, troponin C and S100B. These proteins typically undergo a calcium-dependent conformational change which opens a target binding site. The latter group is represented by calbindin D9k which remains in a closed conformation upon calcium binding.

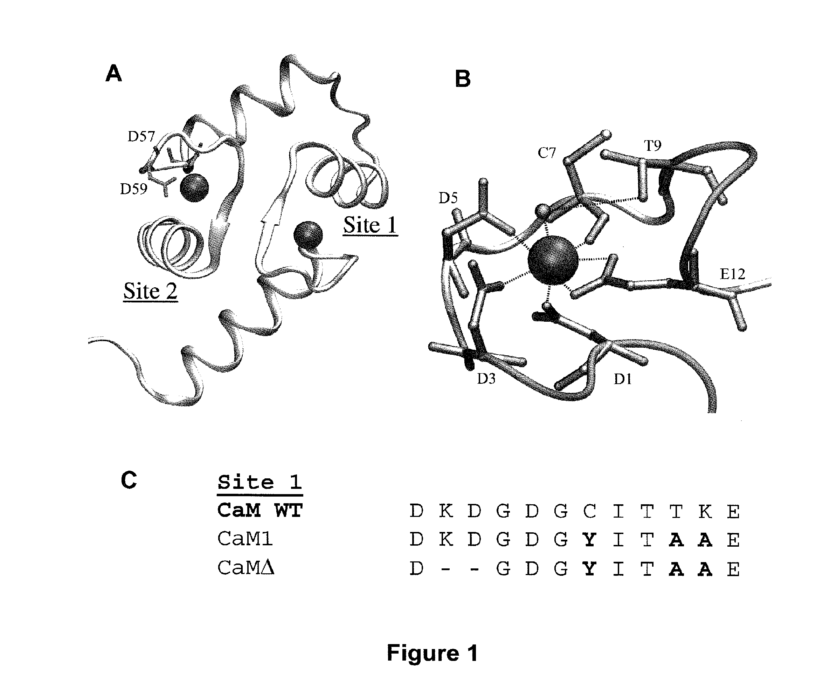

Calmodulin (CaM) is the most studied representative of the ubiquitous EF-hand protein family and a calcium binding protein involved in the regulation of a wide range of target enzymes. The calmodulin structure (PDB code 1EXR) includes two pairs of EF-hand motifs (EF-hands 1 and 2; EF-hands 3 and 4) in two domains (domain 1 and 2) separated by a flexible α-helix. In EF-hand1, calcium ligands are provided by three monodentate aspartate (D) at positions 1, 3, 5 of the metal-binding loop (D1, D3, D5), a bidentate glutamate at position 12 (E12), a main chain carbonyl at position 7 and a water molecule stabilized by threonine 9 (T9) side chain, as schematized in Figure 1B.

Fluorescent sensors for calcium, called cameleons, have been constructed based on green fluorescent proteins and calmodulin (Miyawaki et al., Nature, 1997, 388, 882-887; Nagai et al., PNAS, 2004, 101, 10554-10559). They are chimeric proteins composed of a short-wavelength variant of GFP, CaM, a glycylglycine linker, the CaM-binding peptide of myosin light-chain kinase (M13), and a long wave-length variant of GFP. Ca²⁺ binding to CaM initiates an intramolecular interaction between CaM and M13, which changes the chimeric protein from an extended to a more compact conformation, thereby increasing the efficiency of Fluorescence Resonance Energy Transfer (FRET) from the shorter-to the longer-wavelength variant of GFP. Yellow cameleons (YCs) have cyan and yellow fluorescent proteins (CFP and GFP) as the FRET donor and acceptor, respectively.

Synthetic cyclic-peptide variants of calmodulin site 1 (CaM-Mc peptides), consisting of a 33 amino acid sequence in which one, two or three of the aspartic acid residues in positions 1, 3 and 5 of the calcium-binding loop (D1, D3, D5) are substituted with neutral amino acids (T, N or S) and a tyrosine residue is introduced in position 7 of the loop to monitor metal binding using tyrosine fluorescence bind uranyl with an apparent dissociation constant in the micromolar range (K_{d} of 9.8 to 54.10⁻⁶ M) while they don't bind calcium (WO 2005/012336). However, uranyl biosensors with detection limits in the nanomolar range or below are required to detect uranium content in waters at the maximum concentration levels defined by the international radioprotection authorities.

A recombinant phosphorylated variant of calmodulin domain 1, CaM1P, in which the threonine at position 9 of the EF-hand1 loop is phosphorylated, was shown to bind uranyl with a dissociation constant in the subnanomolar range, at pH 7 (K_{d} of 3.10⁻¹⁰ M; Pardoux et al., 2012 PLoS ONE, 2012, 7, e41922). CaM1P is derived from CaM1 which consists of a 77 amino acids sequence, in which: (i) the T₉TKE₁₂ sequence of EF-hand1 loop is substituted by the CK2 recognition sequence TAAE to allow phosphorylation of the threonine 9, *in vitro,* using recombinant catalytic subunit of protein kinase CK2, (ii) a tyrosine residue is introduced in position 7 of the EF-hand 1 loop so that uranyl and calcium binding affinities could be determined by following tyrosine fluorescence emission at 302 nm, and (iii) the metal binding ability of site 2 is impaired by substituting the two aspartate residues in positions 1 and 3 of the loop with alanine residues.

Phosphorylation of the threonine at position 9 of the metal-binding loop increased uranyl binding affinity by a factor of 5 at pH 6, while increasing the pH to 7 led to a further enhancement in uranyl affinity by a factor of 15.6. Analysis of the infrared modes of the phosphoryl group indicated that this group was deprotonated at pH 7 and directly involved in uranyl coordination.

However, CaM1P affinity for calcium (∼ 20.10^{-6M}) is similar to that of CaM domain 1 so that its selectivity for uranyl over calcium is low (4.10³ at pH 6 and 6.10⁴ at pH 7).

In addition CaM1P cannot be expressed in a recombinant cell, microorganism or plant, which is then used for the *in situ* biodetection or bioremediation of uranyl because the phosphorylation of the Threonine 9 is performed *in vitro.*

In this context, new sensitive and specific metal biosensors and/or chelators would be useful for the development of cost-effective uranium biodetection and bioremediation strategies.

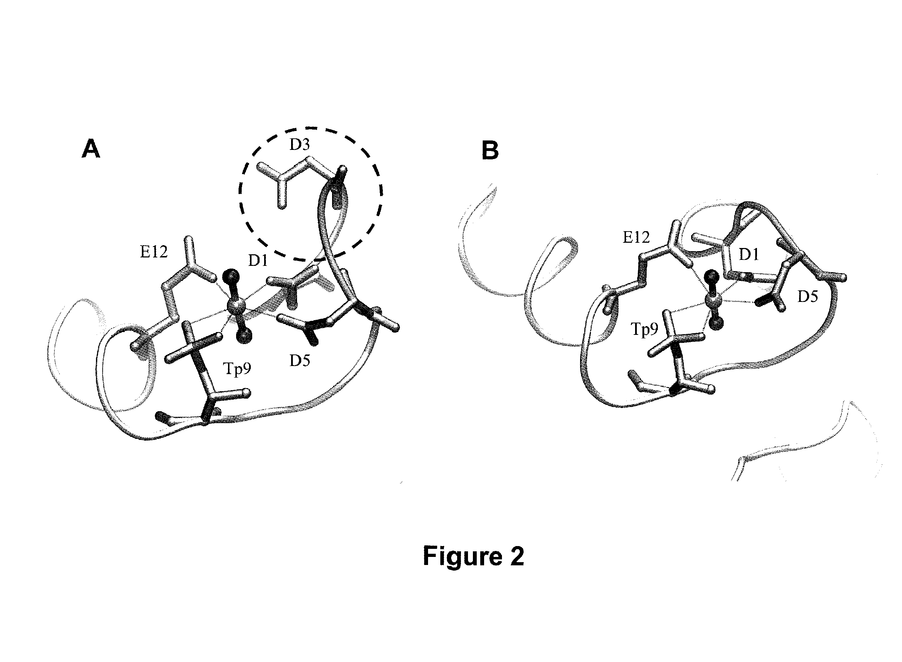

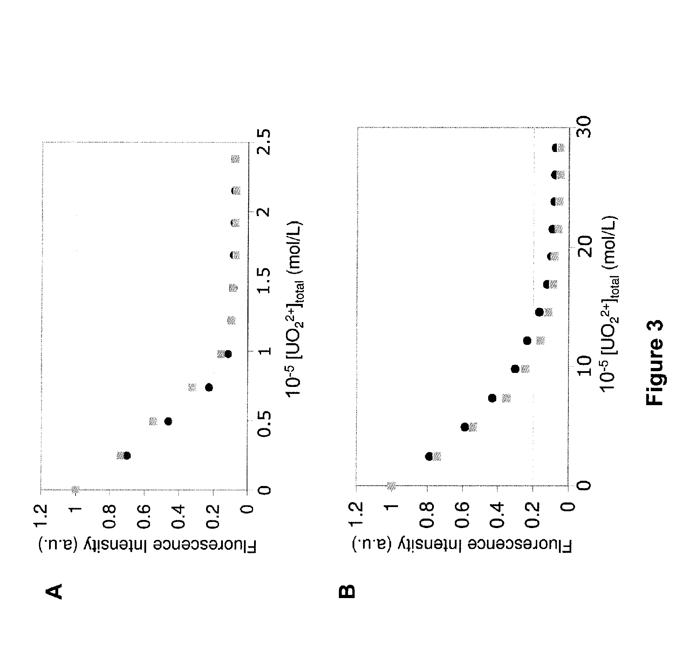

The inventors have determined a structural model of the complex formed by the phosphorylated CaM1 peptide (CaM1P) with uranyl using a molecular dynamics approach. This structural model suggested that at least two residues of the calcium-binding loop are not necessary to bind uranyl in the complex: at least one of the calcium-ligating residues (aspartate at position 3 or D3) and its directly adjacent residue (Lysine at position 2 or K2) (Figure 2A). This result encouraged the inventors to produce a variant CaMΔ by the deletion of these two residues of the loop (K2 and D3) formerly to analyse the resulting properties of the phosphorylated peptide CaMΔ-P (Figure 2B). Unexpectedly, the non phosphorylated peptide CaMΔ showed a very high affinity for uranyl, and a low affinity for calcium, as demonstrated in the examples of the present application (Figure 3).

One aspect of the present invention relates to a polypeptide comprising at least one helix-loop-helix calcium-binding (EF-hand) motif, which comprises a deletion of at least one amino acid residue in the 12-amino-acid calcium-binding loop sequence, and wherein said polypeptide binds uranyl.

The polypeptide of the invention which is an isolated recombinant or synthetic polypeptide, has a signicantly higher binding affinity for uranyl and a significantly lower binding affinity for calcium than the corresponding peptide without said deletion. In the present application, "significant" means that the binding affinity is different with P<0.01. Binding affinity for metals can be measured by any standard technique which is known by those skilled in the art such as those described in the examples of the present application.

Preferably, the polypeptide of the invention has a binding affinity for uranyl which is increased by a factor of at least 2, more preferably at least 5, 10, 30, 100 or more and a binding affinity for calcium which is decreased by a factor of at least 2, more preferably at least 5, 10, 30, 100, 300 or more compared to the corresponding peptide without the deletion in the calcium-binding loop. Preferably, the polypeptide of the invention has a selectivity for uranyl over calcium which is of at least 10³, more preferably at least 10⁴, 10⁵, 10⁶, 10⁷ or more.

For example, the CaMΔ peptide of the examples has a binding affinity for uranyl which is increased by a factor of at least 100 and a binding affinity for calcium which is decreased by a factor of at least 500 compared to the corresponding peptide CaM1 which does not have the deletion in the calcium- binding loop. Therefore the selectivity of CaMΔ for uranyl over calcium is of the order of 10⁷ whereas that of CaM1 is only of the order of 10³.

The polypeptide of the invention and its derivatives like the cameleon-based biosensors, have the following advantages:
- they have a high binding affinity for uranyl combined with a low binding affinity for calcium, which means that they are highly sensitive and selective for uranyl. Therefore, they can detect low uranium concentration in complex media containing divalent cations like Ca²⁺ (for example, biological media, calcium-rich water). Uranyl detection in a sample to analyze can be performed *in vitro* using the isolated polypeptide or cameleon-based biosensor, as well as *in situ* in recombinant cells (whole-cell biosensors) or non-human transgenic organisms expressing the cameleon protein derived from the polypeptide of the invention. Their affinity and selectivity for uranium are higher than those of all calmodulin-derived uranium biosensors.
- the cameleon biosensors derived from the polypeptide of the invention can also be used as imaging biosensor to visualize uranyl *in situ* in recombinant cells or non-human transgenic organisms expressing the cameleon protein derived from the polypeptide of the invention,
- the recombinant cells and non-human transgenic organisms expressing the polypeptide or its derivatives can be used for the biodecontamination and the bioremediation of uranium contamination in an environment, as well as for the production of large quantities of the polypeptide of the invention and its derivatives (cameleon-based biosensors,..).
- as recombinant proteins, their production and use are easy, fast not-toxic, and cost-effective, and they are designed for *in vitro* and *in vivo* uses.

In the following description, the standard one letter amino acid code is used. The expression "EF-hand motif" refers to the canonical EF-hand calcium-binding motif as described just before.

In one embodiment, the polypeptide comprises a deletion of at least one of the calcium-ligating residues (residues in positions 1, 3, 5, 7, 9 and/or 12 of the calcium-binding loop sequence). Preferably, the polypeptide comprises a deletion of at least one of the calcium-ligating residues in positions 1, 3, and/or 5 of the calcium-binding loop sequence. More preferably, the polypeptide comprises a deletion of the calcium-ligating residue in position 3 of the calcium-binding loop.

In another embodiment, the polypeptide comprises a deletion of at least two amino acid residues in the calcium-binding loop sequence. Preferably, the polypeptide comprises a deletion of at least one calcium-ligating residue and its directly adjacent residue (*i.e*., residue in position +1 or -1 relative to the calcium-ligating residue). More preferably, the polypeptide comprises a deletion of at least one of the following pairs of residues: positions 1 and 2, 2 and 3, 3 and 4, 4 and 5, 5 and 6 of the calcium-binding loop. Even more preferably, the polypeptide comprises a deletion of at least the pair of residues in positions 2 and 3 of the calcium-binding loop.

In another preferred embodiment, the polypeptide comprises at least two EF-hand motifs, wherein at least one EF-hand motif comprises said deletion in the loop sequence and the other EF-hand motif(s) comprise said deletion in the loop sequence or not. Preferably, the polypeptide comprises two to four EF-hand motifs. More preferably, the polypeptide comprises two or four EF-hand motifs. All the EF-hands motifs of the polypeptide are advantageously from the same EF-hand protein.

In another preferred embodiment, the polypeptide comprises further alterations in said EF-hand motif(s) which comprise said deletion or not, such as for example the substitution and/or the modification of one or more amino acid residues. Said alterations are introduced when the amino acid residues of interest are not naturally present at the appropriate positions in said EF-hand motif(s). Preferably, said further alterations are in the loop sequence.

In a first advantageous arrangement of said embodiment, the polypeptide comprises a tyrosine residue in position 7 of the loop from said EF-hand motif (s) which comprise said deletion or not, by reference to the numbering of the 12-amino-acid loop sequence. Preferably, said tyrosine residue is in the loop from said at least one EF-hand motif which comprises said deletion.The tyrosine in position 7 allows the monitoring of uranyl and/or calcium binding and the determination of their binding affinities, by following tyrosine fluorescence emission at 302 nm. The tyrosine in position 7 is naturally present in the EF-hand motif(s) or substituted.

In a second advantageous arrangement of said embodiment, the polypeptide comprises at least two aspartic acid residues in positions 1, 3 and/or 5 of the loop from said at least one EF-hand motif which comprises said deletion, by reference to the numbering of the 12-amino-acid loop sequence.

In a third advantageous arrangement of said embodiment, the polypeptide comprises at least one phosphorylated serine or threonine residue. The phosphorylated serine or threonine residue are advantageously in the loop from said EF-hand motif(s) which comprise said deletion or not, preferably in position 9 and/or 12 of the loop from said EF-hand motif(s), by reference to the numbering of the 12-amino-acid loop sequence. The phosphorylation of said residue(s) increases the uranyl binding affinity of the polypeptide. The residues in positions +1 to +3 relative to the phosphorylated threonine are advantageously modified to provide a CK2 recognition sequence TXXE, in which X is a neutral or acidic amino acid different from T, for example XX is AA or AE. Phosphorylation of the threonine may be performed *in vitro,* using recombinant catalytic subunit of protein kinase CK2. Preferably, the polypeptide comprises a phosphorylated threonine residue in position 9 of the loop from said EF-hand motif(s) which comprise(s) said deletion or not and further comprises alanine residues in positions 10 and 11 of said loop, by reference to the numbering of the 12-amino-acid loop sequence. These alanine residues provide a CK2 recognition sequence TAAE to allow phosphorylation of the Threonine 9. The phosphorylated serine or threonine residues are advantageously in the loop from said at least one EF-hand motif which comprises said deletion. The serine and threonine residues of the EF-hand motif(s) which are modified by phosphorylation and the adjacent residues which are substituted to provide a CK2 recognition site are naturally present in the EF-hand motif(s) or substituted.

In another advantageous arrangement of said embodiment, the polypeptide comprises at least two EF-hand motifs, at least one comprising said deletion in the loop sequence and at least another one not comprising said deletion, wherein at least one of said EF-hand motif(s) not comprising the deletion comprises at least one mutation in the loop sequence which impairs calcium binding. Preferably, said mutation is the substitution with alanine residues of the residues in positions 1 and 3 of the loop by reference to the numbering of the 12-amino-acid loop sequence.

The EF-hand motif sequence is modified by standard mutagenesis technique on the polypeptide coding sequence. The phosphorylation of the serine and/or threonine residues is performed using standard methods which are known from those skilled in the art.

The amino acid-sequence of the EF-hand motif(s) of the polypeptide of the invention is that of the corresponding wild-type EF-hand protein(s) except at said amino acid position(s) which are altered in the present invention.

The polypeptide of the invention may be derived from EF-hand motif(s) of any proteins of the EF-hand family (EF-hand protein) having a canonical EF-hand motif as above described. Preferably, the polypeptide of the invention is derived from EF-hand protein(s) from the class of EF-hand signaling proteins, *i.e*., the EF-hand proteins which undergo a calcium-dependent conformational change. More preferably, it is derived from EF-hand signaling protein(s) of the calmodulin superfamily. Even more preferably, from EF-hand signaling protein(s) of the calmodulin superfamily selected from the group consisting of calmodulin and troponin C. It is advantageously derived from the EF-hand1, 2, 3 and/or 4 of calmodulin, and said EF-hand1, 2, 3 and/or 4 are advantageously from the same calmodulin protein.

In an advantageous arrangement of said embodiment, the polypeptide is derived from *Arabidopsis thaliana* calmodulin 3 (amino acid sequence SEQ ID NO: 2 encoded by the cDNA of SEQ ID NO: 1).

The calmodulin EF-hand1, 2, 3 and 4 correspond respectively to positions 12 to 41, 48 to 76, 85 to 114, and 121 to 149, by reference to the amino acid numbering of *Arabidopsis thaliana* calmodulin 3 (SEQ ID NO: 2). The calcium-binding loop of EF-hand1, 2, 3 and 4 correspond respectively to positions 21 to 32, 57 to 68, 94 to 105, and 130 to 141, by reference to the amino acid numbering of SEQ ID NO: 2. The calmodulin domain 1 and 2 correspond respectively to positions 1 to 76 and 77 to 149, by reference to the amino acid numbering of SEQ ID NO: 2.

In another advantageous arrangement of said embodiment, the polypeptide comprises a EF-hand motif derived from a calmodulin EF-hand1 having a 12 amino-acid loop of sequence (I):
X₁-X₂-X₃-X₄-X₅-X₆-X₇-X₈-X₉-X₁₀-X₁₁-X₁₂, in which:
   X₁ is D; X₂ is K or R, preferably K; X₃ is D; X₄ is G, N, Q, preferably G; X₅ is D or N, preferably D; X₆ is G; X₇ is T, C, S or N, preferably, T; X₈ is I; X₉ is T or S, preferably T; X₁₀ is T or S, preferably T; X₁₁ is K, S, M or N, preferably K, and X₁₂ is E. More preferably, said EF-hand1 has a 12 amino-acid loop of sequence DKDGDGCITTKE (SEQ ID NO: 3).

In another advantageous arrangement of said embodiment, the polypeptide comprises a EF-hand motif derived from a calmodulin EF-hand2 having a 12 amino-acid loop of sequence (II):
X₁-X₂-X₃-X₄-X₅-X₆-X₇-X₈-X₉-X₁₀-X₁₁-X₁₂, in which:
   X₁ is D; X₂ is A, V or Q, preferably A; X₃ is D; X₄ is G, N; X₅ is N, D or S, preferably N; X₆ is G or H, preferably, G; X₇ is T, N or Q, preferably, T; X₈ is I; X₉ is D or E, preferably D; X₁₀ is F; X₁₁ is P, T or S, preferably P, and X₁₂ is E. More preferably, said EF-hand2 has a 12 amino-acid loop of sequence DADGNGTIDFPE (SEQ ID NO: 4).

In another advantageous arrangement of said embodiment, the polypeptide comprises a EF-hand motif derived from a calmodulin EF-hand3 having a 12 amino-acid loop of sequence DKDQNGFISAAE (SEQ ID NO: 5)

In another advantageous arrangement of said embodiment, the polypeptide comprises a EF-hand motif derived from a calmodulin EF-hand4 having a 12 amino-acid loop of sequence DVDGDGQINYEE (SEQ ID NO: 6)

A more preferred polypeptide of the invention is derived from a calmodulin EF-hand1, EF-hand2, EF-hand3 or EF-hand 4 and comprises a calcium-binding loop sequence having a deletion of at least one amino acid, which is selected from the group consisting of the sequences: DGDGCITTKE (SEQ ID NO: 7), DGDGYITTKE (SEQ ID NO: 8), DGDGYITAAE (SEQ ID NO: 9), DGNGTIDFPE (SEQ ID NO: 10), DGNGYIDFPE (SEQ ID NO: 11), DGDGTIDFPE (SEQ ID NO: 12) DGDGYIDFPE (SEQ ID NO: 13), DQNGFISAAE (SEQ ID NO: 14) and DGDGQINYEE (SEQ ID NO: 15).

In another advantageous arrangement of said embodiment, the polypeptide of the invention is a calmodulin domain 1 variant comprising two EF-hand motifs, respectively from the EF-hand1 and the EF-hand2 of the same calmodulin protein. Preferably the EF-hand1 comprises the deletion of the amino acid residues in positions 2 and 3, a tyrosine residue in position 7, and neutral or acidic residues different from T in positions 10 and 11 of the loop, for example AA or AE, and the EF-hand2 comprises the substitution of the residues in positions 1 and 3 of the loop with alanine residues, which impairs metal binding in site 2. More preferably, said polypeptide comprises or consists of the sequence SEQ ID NO: 17, which corresponds to the peptide referred to as CaMΔ in the examples of the present application. CaMΔ is a variant of the domain 1 from *Arabidopsis thaliana* calmodulin 3.

In another advantageous arrangement of said embodiment, the polypeptide of the invention is a calmodulin variant comprising four EF-hand motifs, respectively from the EF-hand1, 2, 3 and 4 of the same calmodulin protein.

In one preferred polypeptide, the EF-hand1 comprises the deletion of the amino acid residues in positions 2 and 3 and the EF-hand2 comprises no deletion. More preferably, the EF-hand2 further comprises the substitution of the residues in positions 1 and 3 of the loop with alanine residues, which impairs metal binding in site 2. Even more preferably, said polypeptide comprises or consists of the sequences SEQ ID NO: 18 and 19 derived from *Arabidopsis thaliana* calmodulin 3 which were used for the construction of the cameleon biosensors delta and delta-S2M in the examples of the present application.

In another preferred polypeptide, each of the EF-hand1, 2, 3 and 4 comprise the deletion of the amino acid residues in positions 2 and 3. Even more preferably, said polypeptide comprises or consists of the sequence SEQ ID NO: 20 which is derived from *Arabidopsis thaliana* calmodulin 3.

In another preferred embodiment, the polypeptide is coupled to a labeling agent which produces a detectable and/or quantifiable signal, in particular a radioactive, magnetic or luminescent (radioluminescent, chemiluminescent, bioluminescent, fluorescent or phosphorescent) agent. The labeled polypeptide may be labeled directly or indirectly, via covalent or non-covalent bonds, using standard conjugation techniques that are well-known to those skilled in the art.

Another aspect of the present invention relates to a fusion or chimeric protein comprising the polypeptide fused to another protein moiety, directly or through a peptide spacer. The protein/peptide moieties include those which allow the purification, detection, immobilization, and/or cellular targeting of the polypeptide of the invention, and/or which increase the affinity for uranyl, the bioavailability, and/or the production in expression systems of said polypeptide. These moieties may be selected from: (i) a labeling moiety such as a fluorescent protein (GFP and its derivatives, BlueFP, CyanFP, YellowFP, GreenFP and red-shifted GFP), (ii) a reporter moiety such as an enzyme tag (luciferase, alkaline phosphatase, *glutathione-S-transferase* (GST), β-galactosidase), (iii) a binding moiety such as an epitope tag (polyHis6, FLAG, HA, myc.), a DNA-binding domain, a hormone-binding domain, a poly-lysine tag for immobilization onto a support, and (iv) a targeting moiety for addressing the chimeric protein to a specific cell type or cell compartment. In addition, the sequence(s) advantageously comprise a linker which is long enough to avoid inhibiting interactions between said sequence(s) and the polypeptide sequence. The linker may also comprise a recognition site for a protease, for example, for removing affinity tags from the purified chimeric protein according to the present invention.

In a preferred embodiment the chimeric protein is a cameleon protein comprising tandem fusions of (1) the polypeptide of the invention, (2) a EF-hand protein binding-peptide that binds a complex between the polypeptide in (1) and calcium, (3) a fluorescence-donor protein, and (4) a fluorescence-acceptor protein, wherein the fluorescence donor and acceptor proteins are at each end of the cameleon protein.

The polypeptide and the EF-hand protein binding peptides are advantageously separated by a linker. The linker is preferably of 2 to 10 amino acids, which may be G and/or S residues or random amino acid residues.

The fluorescence donor and acceptor proteins which are advantageously at each end of the cameleon protein are chosen from any protein capable of producing FRET such as with no limitation: Alexa proteins and GFP variants such as those disclosed for example in Shaner et al., Nature Methods, 2005, 12, 905-909; Erard et al., Molecular Biosystems, 2013, 9, 258-267; Fredj et al., PLOS ONE, 2012, 7, e49149 and PCT Application WO/2012/172095.

The fluorescence donor and acceptor proteins are advantageously chosen from a short-wavelength variant of GFP such as CyanFP or BlueFP, Turquoise, Turquoise 2, mutated *Av*CFP, Cerulean, Cerulean3, TFP1 and a long wave-length variant of GFP such as YellowFP, GreenFP and red-shifted GFP, Citrine, Venus and circularly permuted fluorescent proteins.

The polypeptide of the invention comprises advantageously four EF-hand motifs from the same EF-hand signaling protein as defined above. Preferably said EF-hand motifs are from a EF-hand signaling protein of the calmodulin superfamily selected from the group consisting of calmodulin and troponin C.

In an advantageous arrangement of said embodiment, the cameleon protein is derived from calmodulin.

Many peptides which bind calmodulin in complex with calcium are known in the art (see for example Carafoli et al., Proc. Natl. Acad. Sci. USA., 2002, 99 :1115-1122). Any of these peptides can be used in the cameleon protein of the invention, including with no limitations: the peptides M13 (amino acid sequence SEQ ID NO: 22 encoded by the nucleotide sequence SEQ ID NO: 21) and skMLCK (SEQ ID NO: 23) from skeletal myosin light chain kinase; the peptides MLCKp (SEQ ID NO: 24) and smMLCK (SEQ ID NO: 25) from myosin light chain kinase; the peptide named wasp venom ou polistes mastoparan (SEQ ID NO: 26); the peptide p21 (SEQ ID NO: 27); the peptides disclosed in Shifman J.M. and Mayo, L., PNAS, 2003, 100, 13274- such as melittin (SEQ ID NO: 28), spectrin (SEQ ID NO: 29), CaMKI (SEQ ID NO: 30), CaMKII (SEQ ID NO: 31), CaMKK (SEQ ID NO: 32) and peptide1(SEQ ID NO: 33); the peptides from cyclic nucleotide phophodiesterase (Olwin & Storm, 1985); the peptides from caldesmon (Yazawa *et al.,* 1987) and the synthetic peptide derived from the plasma membrane Ca²⁺ pump (Yazawa *et al.,* 1992).

Preferably, the cameleon protein comprises a short-wavelength variant of GFP such as CyanFP or BlueFP, a polypeptide of the invention derived from calmodulin, a linker, the calmodulin-binding peptide of myosin light chain kinase (peptide M13), and a long wave-length variant of GFP such as YellowFP, GreenFP and red-shifted GFP. One preferred polypeptide is a calmodulin variant comprising a EF-hand1 with said deletion and a EF-hand2 with no deletion. More preferably, the EF-hand2 comprises a mutation which impairs metal binding. Another preferred polypeptide is a calmodulin variant wherein each of the EF-hand1, 2, 3 and 4 comprise said deletion.

Preferred calmodulin-derived cameleon proteins of the invention comprise or consist of an amino acid sequence selected from the group consisting of the sequences SEQ ID NO: 35, 37 and 38.

Alternatively, the four elements of the cameleon protein as described above can be divided in two separate fusion protein, which are then combined together to obtain a functional biosensor capable of detecting uranium: a first fusion protein with the fluorescence donor fused to one of the polypeptide or the polypeptide binding-peptide, and a second fusion protein with the fluorescence acceptor fused to the polypeptide or polypeptide binding-peptide which is not fused to the fluorescence donor. These types of cameleon proteins are described for example in Miyawaki et al., Proc. Natl. Acad. Sci. U S A., 1999, 96, 2135-40.

The cameleon protein is a uranyl biosensor that can be used *in vitro* as uranyl analysis reagent for the detection of uranyl in an environment (water, soil, effluents) or in biological samples from individuals (biological fluids). It is also used as cell imaging reagent or diagnostic reagent for the detection of uranyl *in situ* in recombinant cells (whole-cell biosensor) or non-human transgenic organisms expressing the cameleon protein.

The invention encompasses polypeptides and derived fusion proteins comprising or consisting of natural amino acids (20 gene-encoded amino acids in a Land/or D-configuration) linked via a peptide bond as well as peptidomimetics of such protein where the amino acid(s) and/or peptide bond(s) have been replaced by functional analogues. Such functional analogues include all known amino acids other than said 20 gene-encoded amino acids. A non-limitative list of non-coded amino acids is provided in Table 1A of US 2008/0234183 which is incorporated herein by reference. The invention also encompasses modified polypeptides/fusion proteins derived from the above polypeptides/fusion proteins by introduction of any modification into one or more amino acid residues, peptide bonds, N-and/or C-terminal ends of the protein, as long as the uranyl binding activity is maintained in the modified polypeptide/protein. These modifications which are introduced into the polypeptide/protein by the conventional methods known to those skilled in the art, include, in a non-limiting manner: the substitution of a natural amino acid with a non-proteinogenic amino acid (D amino acid or amino acid analog); the modification of the peptide bond, in particular with a bond of the retro or retro-inverso type or a bond different from the peptide bond; the cyclization, and the addition of a chemical group to the side chain or the end(s) of the protein, in particular for coupling an agent of interest to the polypeptide/fusion protein of the invention.

In another preferred embodiment, the polypeptide or fusion protein is immobilized on the surface of a solid support, such as with no limitation, a plate, a slide, a strip, a fiber, a gel, a felt support, wells, microparticles, or biologically modified ceramics (biocers; Böttcher et al., J. Mater. Chem., 2004, 14, 2176-2188).

Another aspect of the invention relates to an isolated polynucleotide encoding a polypeptide or chimeric protein of the invention. The synthetic or recombinant polynucleotide may be DNA, RNA or combination thereof, either single- and/or double-stranded. Preferably the polynucleotide comprises a coding sequence which is optimized for the host in which the polypeptide or chimeric protein is expressed. In a preferred embodiment, the polynucleotide comprises or consists of a sequence selected from the group consisting of the sequences SEQ ID NO:16, 34 and 36.

Another aspect of the invention relates to a recombinant vector comprising said polynucleotide. Preferably, said recombinant vector is an expression vector capable of expressing said polynucleotide when transfected or transformed into a host cell such as a mammalian, bacterial or fungal cell. The polynucleotide is inserted into the expression vector in proper orientation and correct reading frame for expression. Preferably, the polynucleotide is operably linked to at least one transcriptional regulatory sequence and, optionally to at least one translational regulatory sequence. In a preferred embodiment, the polynucleotide is under the control of a promoter which is upregulated in response to uranium such as for example *Caulobacter urc-A* promoter (Hillson et al., Applied and Environmental Microbiology, 2007, 73, 7615-7621). Recombinant vectors include usual vectors used in genetic engineering and gene therapy including for example plasmids and viral vectors.

Another aspect of the invention provides a host cell or a non-human organism transformed with said polynucleotide or recombinant vector. Preferably, said modified host cell or non-human transgenic organism is resistant to radiations, and/or pollutants such as for example nitrates and toxic metals. The non-human transgenic organism is obtained from a unicellular or pluricellular microorganism or a higher eukaryotic organism. In a preferred embodiment said modified host cell is a prokaryotic cell such as a bacteria. In another embodiment, said non-human transgenic organism is a transgenic plant, nematode, zebrafish or algae.

The polynucleotide, vector, cell, and non-human transgenic organism of the invention are useful for the production of the polypeptide/chimeric protein of the invention using well-known recombinant DNA techniques.

Another aspect of the invention relates to the use of the polypeptide, fusion protein, host cell, non-human transgenic organism, *in vitro* or *in vivo*, as uranyl chelating agent. The chelating agent is useful for the detection, the decontamination, and/or the bioremediation of uranium contamination in an environment (water, soil, effluents,...) or in individuals.

The labeled polypeptide/fusion protein such as the polypeptide comprising a tyrosine residue in position 7 of the loop or the cameleon protein, is a uranyl biosensor. It can be used *in vitro* as uranyl analysis or diagnostic reagent for the detection of uranyl in an environment (water, soil, effluents,...) or in biological samples from individuals (biological fluids,..). In addition, the cameleon protein is also used as cell imaging reagent, diagnostic reagent and bioindicator for the detection of uranyl *in situ* in modified cells (for example recombinant cells) or non-human transgenic organisms expressing the cameleon protein.

The polynucleotide according to the invention is prepared by the conventional methods known in the art. For example, it is produced by amplification of a nucleic sequence by PCR or RT-PCR, by screening genomic DNA libraries by hybridization with a homologous probe, or else by total or partial chemical synthesis. The recombinant vectors are constructed and introduced into host cells by the conventional recombinant DNA and genetic engineering techniques, which are known in the art.

The polypeptide/chimeric protein is prepared by the conventional techniques known to those skilled in the art, in particular by solid-phase or liquid-phase synthesis or by expression of a recombinant DNA in a suitable cell system (eukaryotic or prokaryotic). More specifically, the polypeptide can be solid-phase synthesized, according to the Fmoc technique, originally described by Merrifield et al. (J. Am. Chem. Soc., 1964, 85: 2149-), and purified by reverse-phase high performance liquid chromatography; the polypeptide/chimeric protein can be produced from the corresponding cDNAs, obtained by any means known to those skilled in the art; the cDNA is cloned into a eukaryotic or prokaryotic expression vector and the protein produced in the cells modified with the recombinant vector is purified by any suitable means, in particular by affinity chromatography.

The practice of the present invention will employ, unless otherwise indicated, conventional techniques which are within the skill of the art. Such techniques are explained fully in the literature.

In addition to the above arrangements, the invention also comprises other arrangements, which will emerge from the description which follows, which refers to exemplary embodiments of the subject of the present invention, with reference to the attached drawings in which:
- Figure 1 is a schematic of calmodulin. **A.** Schematic of domain 1 of *Paramecium tetraurelia* calmodulin (PDB code 1 EXR) which comprises two calcium-binding sites (site 1 and site 2) which are part of a canonical EF-hand motif (EF-hand1 and EF-hand2, respectively for site 1 and site 2). **B.** Structural model of the calcium binding site 1 of *Arabidopsis thaliana* calmodulin showing the coordinating amino acids. **C.** Amino acid sequence of calmodulin site 1 from *Arabidopsis thaliana* (CaM-WT) and from the peptide CaM1 and CaMΔ analysed in the present study. In the CaM1 and CaMΔ peptides, the metal binding ability of site 2 was impaired by replacing the aspartate residues in positions 57 and 59 of calmodulin (positions 58 and 60 of CaM1) with alanines. In addition, the threonine residue in position 31 and the lysine residue in position 32 of CaM1 (positions 10 and 11 of site 1) have each been substituted with an alanine residue to obtain an efficient CK2 consensus sequence that targets phosphorylation of threonine 9 (T9) of the metal binding loop (T30 in the peptide) and a tyrosine was introduced at position 28 of peptide CaM1 (position 7 of the metal binding loop) to allow the monitoring of uranyl and calcium binding and the determination of their binding affinities, by following tyrosine fluorescence emission at 302 nm.
- Figure 2 represents structural models obtained by molecular dynamics of the CaM1P-U **(A)** and CaMΔP-U **(B)** complexes. Amino acids D1, D5, Tp9 and E12 interact with uranyl. E=Glutamic acid; D=Aspartic acid; Tp=Phosphothreonine.
- Figure 3 shows the binding thermograms of the CaMΔ and CaMΔP peptides with uranyl at pH 6 **(A)** and pH 7 **(B).** Conditions: 10 µM CaMΔ and CaMΔP, 100 µM IDA, 20 mM MES and 100 mM KCl. Full square: CaMΔP. Full circle: CaMΔ.
- Figure 4 shows the binding thermograms of the CaMΔ with uranyl in the absence (empty triangle) and in presence (empty circle) of 10 mM CaCl₂ Conditions: 10 µM CaMΔ, 100 µM IDA, 20 mM MES and 100 mM KCl.
- Figure 5 shows the growth curve of three *E. coli* strains transformed with respectively, the expression vector without insert (as a control) or vectors expressing recombinant CaM1 or CaMΔ peptide, in the presence or absence of uranyl acetate (U). Control (empty circle). Control + U (full circle). CaM1 (empty triangle). CaM1 + U (full triangle). CaMΔ (empty square). CaMΔ + U (full square). IPTG was added at t=105 min; uranyl acetate (U) or sodium acetate were added at t=135 min.
- Figure 6 shows the emission spectra of cameleon biosensor WT protein (excited at 440 nM) at varying CaCl₂ concentrations between 0 µM to 10 µM. 0 µM Ca (full diamond). 2 µM Ca (empty circle). 4 µM Ca (empty triangle). 6 µM Ca (full circle). 8 µM Ca (cross). 10 µM Ca (empty square).
- Figure 7 shows the emission spectra of cameleon biosensor WT protein (excited at 440 nM) at varying uranyl nitrate concentrations between 0 µM to 10 µM. 0 µM UO₂ (full circle). 2 µM UO₂ (empty square). 4 µM UO₂ (full triangle). 6 µM UO₂ (empty circle). 8 µM UO₂ (full circle). 10 µM UO₂ (empty square).

### Example 1: Construction and characterization of calmodulin peptide CaMΔ

### 1. Methods

The recombinant peptides were produced in *E*.*coli.* A histidine-tag followed by the Tobacco Etch Virus protease (TEV) recognition sequence was introduced at the N-terminus, allowing the purification of the peptides using two subsequent chromatography steps on Ni-columns.

### 1.1 engineering and purification of calmodulin derived peptides

The CaM1 construct containing the *Arabidopsis thaliana* sequence of calmodulin domain 1 was obtained as previously described (Pardoux et al., PLoS One, 2012, 7, e41922) and used as a template for new constructs. The CaM1 construct (nucleotide sequence SEQ ID NO: 39 /amino acid sequence SEQ ID NO: 40) comprises the following mutations, by reference to CaM1 amino acid sequence: (1) C28Y mutation to allow the monitoring of uranyl and calcium binding and the determination of their binding affinities, by following tyrosine fluorescence emission at 302 nm, (2) T31A and K32A mutations to enable efficient phosphorylation of T30 by CK2, and (3) D58A and D60A mutations to inactivate the metal binding site 2 of domain 1.

To obtain CaMΔ construct (nucleotide sequence SEQ ID NO: 16/amino acid sequence SEQ ID NO: 17), deletions of K23 and D24 were produced with the QuickChange site-directed mutagenesis kit (STRATAGENE) and specific primer pairs DGD S (SEQ ID NO: 41) and DGD AS (SEQ ID NO: 42), according to the manufacturer's instructions. The engineering plasmid was called pQE-CaMΔ.

Recombinant fusion proteins expressed in *E*. *coli* strain M15Rep4 (QIAGEN) were grown at 37°C in LB medium containing ampicillin (50 µg/mL) and kanamycin (50 µg/mL). Expression was induced by addition of 0.1 mM isopropyl-D-thiogalactoside once OD₆₀₀ reached 0.5, and the cultures were further incubated for 5 h at 37°C. Cellular extracts were obtained by French press lysis and a centrifugation step of 30 min at 15000 rpm, and were applied at a 1 mL/min flow rate on a 5 mL HiTrap Chelating Column (GE HEALTHCARE) in buffer A (50 mM Tris-HCl, 0.5 M NaCl, 25 mM imidazole buffer pH 7.5) containing 1 mM 4-(2-Aminoethyl)benzenesulfonyl fluoride hydrochloride (AEBSF). The proteins were eluted from the nickel resin at a 4 mL/min flow rate using buffer A supplemented with 150 mM imidazole. The proteins were dialyzed against buffer A and the His-Tags were removed by incubation overnight at 4°C with TEV protease, followed by separation using a HiTrap Chelating Column. Recombinant proteins were dialyzed against 50 mM Tris-HCl, 150 mM NaCl, pH 7.5. The protein concentrations were measured according to the BC Assay (UPTIMA) with bovine serum albumin as standard. The proteins were concentrated using the Microcon filtration system (Amicon Millipore^{®}), with a cut-off point of 3 kDa.

Phosphorylation of the peptide CaMΔ was performed as previously described in Pardoux et al., PLoS One, 2012, 7, e41922.

### 1.2 Tyrosine fluorescence titrations

The metal-binding affinity of the various peptides for calcium and uranyl was examined by monitoring the fluorescence intensity of the single tyrosine residue (Tyr28).

The uranyl solutions were prepared extemporaneously by diluting a 0.1 M stock solution of uranyl nitrate (pH 3.5, stored frozen at -20°C) in the final buffer. Fluorescence titrations in the presence of uranyl were performed using a 10 µM peptide solution in MES (20 mM, pH 6) or Tris (20 mM, pH 7) buffer with 100 mM KCl and 100 µM iminodiacetate (IDA). Fluorescence titrations in the presence of calcium were performed in a 10 µM peptide solution in MES (20 mM, pH 6) or Tris (20 mM, pH 7) buffer with 100 mM KCl. To remove any trace of calcium from the samples, each sample solution was incubated 1 h with Chelex®-100 before uranyl or calcium addition.

Spectra were collected on a Cary Eclipse spectrofluorimeter at 25°C, with 270 nm excitation. Emission was observed from 290 to 350 nm. The excitation and emission slits were 10 nm. A 15 min equilibration time was respected before each measurement. The reported stability constants are averages of three experimental values.

Competition experiments between calmoduline-derived peptides CaM peptides and IDA were performed to determine the conditional dissociation constants of the peptide-uranyl complexes at pH 6 and pH 7. IDA has a moderate affinity for uranyl and forms three major complexes: UO₂IDA, [UO₂(IDA)₂]²⁻, and [(UO₂)₂(IDA)₂(OH)₂]²⁻. The conditional stability constants of these three species were calculated from the pKₐₛ and the stability constants at 25°C and 0.1 M ionic strength given by Jiang et al. (Inorg. Chem., 2003, 42, 1233-1240). These three conditional stability constants were fixed in the spectral data analysis, which was performed using the program SPECFIT (Binstead et al., Specfit Global Analysis System Version 3.0.34, 2003). Identical values were obtained for the conditional stability constants of the UO₂-P complexes (where P stands for peptide), either considering that the UO₂-P complex emits or not. In the former case, the spectrum of the UO₂-P complex was calculated to be zero, as the fluorescence emission of tyrosine was totally quenched in the complex.

For titrations in the presence of calcium, the conditional dissociation constants (K_{d}) were determined by fitting the difference between fluorescence intensities measured in the presence (F) and in the absence (F0) of calcium, according to a one site saturation model : ΔF = (Fₘₐₓ x [Ca]) / (Kd + [Ca]) using SigmaPlot 10.0 software (Systat Software). In this equation, Fₘₐₓ corresponds to the maximum of fluorescence determined by the software.

### 1.3 Exposure of E. coli cells producing or not the CaMΔ peptide to uranyl toxicity

E. coli cells were grown overnight in LB-MES 100 mM, pH 5.5. The cells where transferred to a LB (1/10)-glucose (4g/L) medium at pH 4.5, inoculated at 1/100 volume. IPTG was added at a OD₆₀₀ₙₘ=0.4. After 30 minutes, 50 µM uranyl(acetate)₂ or 100 µM Na-acetate was added to the medium. Cell growth was followed by measuring the absorption at 600 nm.

### 2. Results

### 2.1 Binding affinity of the CaMΔ peptide for uranyl

The peptides were prepared at a 10 µM concentration in 20 mM MES pH 6 or Tris pH 7, with 0.1 M KCl and 100 µM IDA. Increasing concentrations of uranyl nitrate were added to the peptide solution, until the peptide to uranyl ratio was approximately 1:4. By using this stoichiometric ratio, the protein samples were not affected by uranyl addition (as monitored by UV-Vis absorption), which is crucial for the interpretation of the results. Addition of uranyl nitrate decreased the fluorescence signal emitted by the single tyrosine present in the peptides at position 7 of the metal binding loop (Figure 3). Tyrosine fluorescence quenching by uranyl has been reported in the literature for other proteins such as transferrin.

Conditional dissociations constants of the peptide - uranyl complexes (Kd) resulting from the competition experiments with IDA were determined at pH 6 and pH 7.

Conditional dissociation constants of 1,8 (± 0,5) 10⁻¹⁰ M and 2 (± 0,1) 10⁻¹⁰ M were calculated at pH 6 and pH 7 for the CaMΔ - uranyl complex. There is no significant effect of pH on the affinity of the peptide CaMΔ for uranyl. The affinity of the CaMΔ peptide is two orders of magnitude greater than that of the CaM1 peptide, possessing a 12 amino acid long binding loop, which has a Kd of 25 10⁻⁹ M at pH 6 (Pardoux et al., PLoS One, 2012, 7, e41922).

The phosphorylated peptide CaMΔP presents similar binding affinities for uranyl, with conditional dissociation constants Kd = 4 (±0,09) 10⁻¹⁰M at pH 6 and Kd = 1,3 (±0,3) 10⁻¹⁰M at pH 7.

Moreover, both peptides have a very low affinity for calcium. Conditional dissociation constants in the millimolar range were observed for the CaMΔ - Ca²⁺ complex at pH 6 (Kd > 1 mM) and at pH 7 (Kd = 8,7 mM). Similar dissociation constants were observed for the phosphorylated peptide CaMΔP with Kd of 7.8 mM at pH 6 and Kd = 4.2 mM at pH 7.

### 2.2 Competition experiments with calcium

The selectivity of the two peptides for uranyl as compared to calcium is of the order of 10⁷. To check if this selectivity is actually observed in a medium containing both uranyl and calcium, the binding isotherm of uranyl was measured in the presence of 10 mM CaCl₂ in the solution.

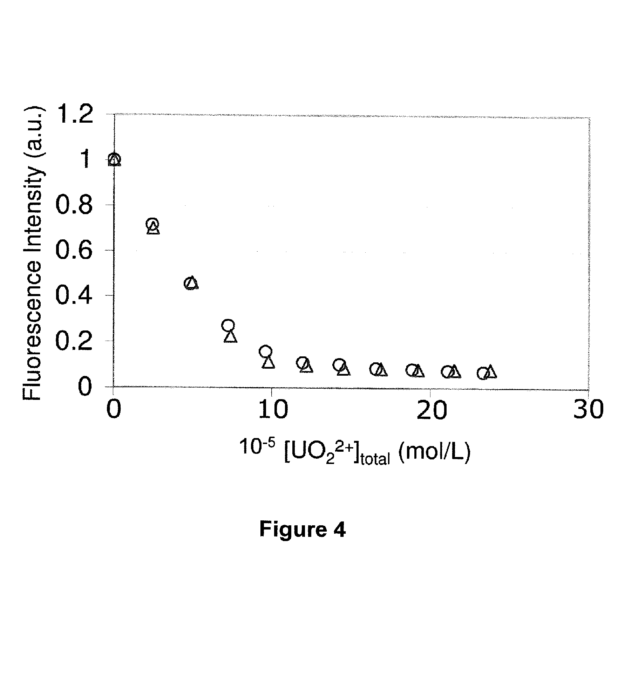

Figure 4 shows the superimposition of binding thermograms corresponding to Tyr fluorescence quenching *(i.e*. uranyl binding) in the absence and in the presence of 10 mM CaCl₂. These results show that calcium has a very modest effect on uranyl titration.

The results of this competition experiment show that these peptides can be used for uranyl detection in the presence of large concentrations of calcium.

### 2.3 The expression of the CaMΔ peptide in E. coli cells decreases uranyl toxicity to the cells

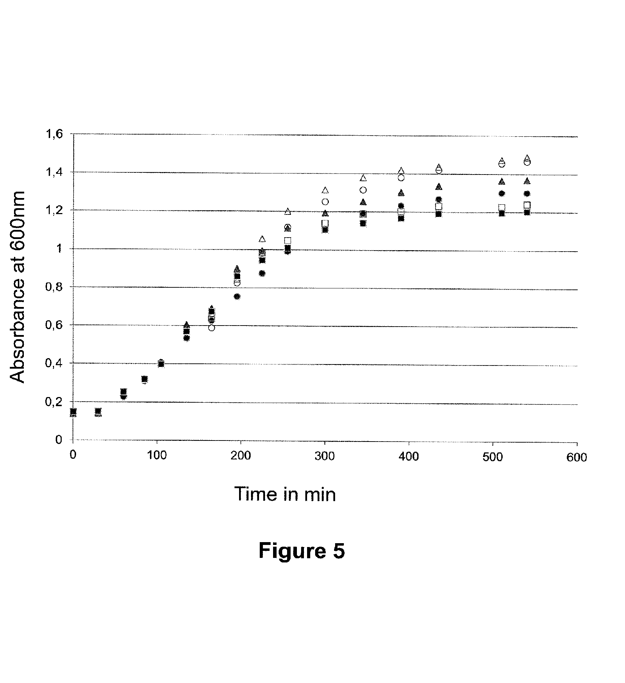

Figure 5 shows growth curves recorded with *E. coli* cells exposed either to 50 µM uranyl acetate, or to 100 µM Na acetate as a control, in LB glucose medium at pH 4.5. Exposure conditions are detailed in the Methods. The figure 5 shows that uranyl exposure stops *E. coli* growth rapidly (full signs as compared to empty signs) except for the cells expressing the CaMΔ peptide. The growth of the cells expressing the CaMΔ peptide is lower than that of the other strains. This may be due to a too strong protein overexpression. However, addition of uranyl didn't affect anymore the growth of this strain. These results suggest that the chelation of uranyl by the CaMΔ peptide protect the whole cells by reducing uranyl toxicity.

### Conclusions

CaMΔ peptide presents an affinity for uranyl in the subnanomolar range and a very high selectivity towards calcium. It is expressed in high quantities in *E. coli* cells.

For these reasons, it is a promising tool for the development of biosensors *(in vivo and in vitro)* or efficient chelating systems *in vitro.*

### Example 2: Construction and characterization of calmodulin derived cameleon biosensors

### 1. Methods

### 1.1 Construction of expression vector for cameleon biosensors

Cloning steps were made with standard methods using XL1Blue cells as *E. coli* strain. All mutations were made using a QuickChange site-directed mutagenesis kit (Stratagene) and specific primer pairs according to the manufacturer.

The gene coding for the cameleon biosensor WT (denoted eCFP-CaM-Linker-M13-eYFP) was constructed in three steps. The gene encoding the wild-type CaM from *A. thaliana* fused with a linker and the CaM-binding peptide of myosin light-chain kinase (M13) was synthetized by Eurofins MWG and cloned into the pQE30 plasmid (QIAGEN) between *Sac* I and *Sal* I restriction sites.

Then, the enhanced Cyan Fluorescent Protein (eCFP) gene containing the TEV protease recognition site upstream of the coding sequence of eCFP was PCR-amplified using the S-TEV-eCFP- *BamH*I (SEQ ID NO: 43) and AS-eCFP-SacI (SEQ ID NO: 44) primers and cloned upstream the CaM-linker-M13 gene, between *BamH* I and *Sac* I restriction sites.

Finally, the enhanced Yellow Fluorescent Protein (eYFP) was PCR-amplified using the S-eYFP- *Sal*I (SEQ ID NO: 45) and AS-eYFP-*Hind*III (SEQ ID NO: 46) primers and cloned downstream the CaM-linker-M13 gene, between the *Sal*I and *Hind* III restriction sites. Both genes contained no stop codon except for the eYFP gene. The cameleon biosensor WT corresponds to the cDNA of SEQ ID NO: 47 and the protein of SEQ ID NO: 48.

The construction of expression vector for the cameleon biosensor Δ was made by using the the cameleon biosensor WT gene as a template and primers S-Δ (SEQ ID NO: 49) and AS-Δ (SEQ ID NO: 50). The constructions of expression vectors for the cameleon biosensor WT-S2M or for the cameleon biosensor Δ-S2M (S2M corresponding to the inactivation of site 2 of the domain I) were made using as a template the cameleon biosensor WT gene or the cameleon biosensor Δ gene respectively and primers S-S2M (SEQ ID NO: 51) and AS-S2M (SEQ ID NO: 52). The cameleon biosensor Δ corresponds to the cDNA of SEQ ID NO: 34 and the protein of SEQ ID NO: 35. The cameleon biosensor WT-S2M corresponds to the cDNA of SEQ ID NO: 53 and the protein of SEQ ID NO: 54. The cameleon biosensor Δ-S2M corresponds to the cDNA of SEQ ID NO: 36 and the protein of SEQ ID NO: 37.

### 1.2 Expression of the cameleon biosensors

The recombinant vectors pQE30 containing the biosensor genes were introduced in the *E. coli* strain M15Rep4. Recombinant fusion proteins were expressed as follows: the overexpression strain was grown at 37°C in LB medium containing ampicillin (50 µg/mL) and kanamycin (50 µg/mL) until OD₆₀₀ reached 0.5. Expression was then induced by addition of 0.1 mM isopropyl-D-thiogalactoside (IPTG) and the cultures were further incubated for 20 h at 17°C. Cells were collected by centrifugation 20 min at 5 000 rpm, and the bacterial pellet was frozen and stored at -80°C.

### 1.3 Purification of the cameleon biosensors

Bacteria were resuspended in buffer A (50 mM Tris-HCl, 0.5 M NaCl, 25 mM imidazole pH 7.5) containing 1 mM 4-(2-Aminoethyl)benzenesulfonyl fluoride hydrochloride (AEBSF) + 15µg/mL DNAse1 + 30 mM MgSO₄. The cellular extracts were obtained by French Press lysis and a centrifugation step of 30 min at 15 000 rpm. The cellular extracts were applied on a 5 mL HiTrap™ Column (GE Healthcare) in buffer A at 1 mL/min flow rate. The proteins were eluted from the nickel resin at 4 mL/min flow rate using an imidazole gradient. The proteins were dialyzed against buffer A and the His-Tags were removed by incubation overnight at 4°C in presence of the TEV protease followed by separation using HiTrap Chelating Column. Gel Filtration was performed for further purification of the proteins using a 26/600 Superdex 200 column (GE HealthCare) and 50 mM Tris-HCl buffer, pH 7.5, supplemented with 150 mM NaCl. The protein concentrations were measured according to the BC Assay from Uptima with bovine serum albumin as standard. The proteins were concentrated using Microcon® filtration system (Amicon Millipore^{®}, with a cut off of 10 kDa).

### 1.4 FRET measurement

Fluorescence experiments were performed using an Infinite 1000 (TECAN). Cameleon biosensor WT proteins were first incubated with an excess of ethylenediaminetetraacetic acid (EDTA) and dialyzed overnight against 50 mM Tris-Cl pH7 containing Chelex resin. This step is used to remove calcium likely to be present in the different CaM binding sites. For each measurement, 1 µM of protein was mixed in 200 µL of 50 mM Tris-Cl pH7 buffer (treated with Chelex) at 25°C. CaCl₂ or uranyl nitrate were added at varying concentrations between 0 and 10 µM. Excitation was performed at 440 nm and the emission spectrum recorded between 450 and 570 nm.

### 2. Results

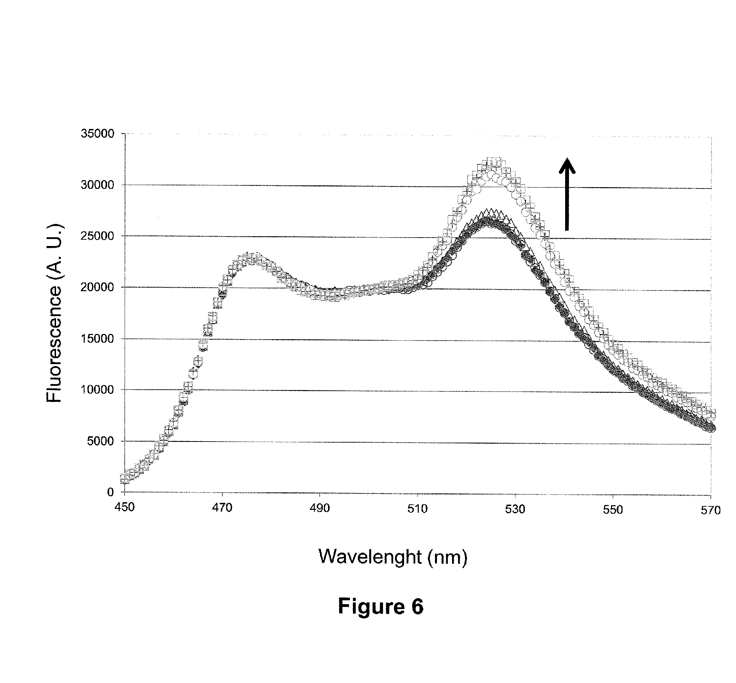

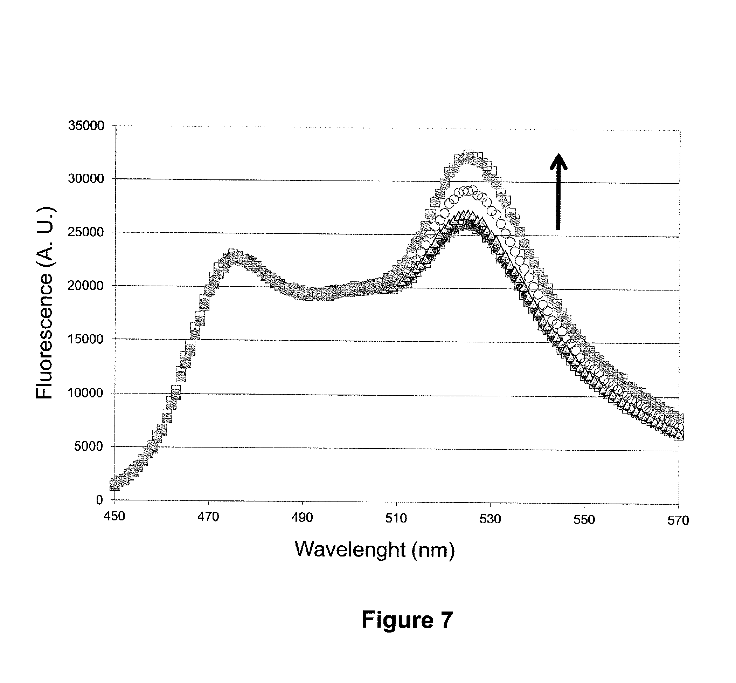

The results obtained with the cameleon biosensor WT protein (Figures 6 and 7, the spectra were normalized at 476 nm) show that this biosensor is able to do some FRET in presence of calcium, and that the maximum of FRET is obtained at 8 µM of calcium and above. Similar results are obtained with 8 µM of uranyl nitrate, showing that the cameleon biosensor obtained with the WT calmodulin shows similar sensitivities for calcium and uranyl.

**TABLE: Amino acid and nucleotide sequences**

| **SEQ ID NO:** | **Name** | **Sequence** |
|---|---|---|
| **1** | *Arabidopsis thaliana* CaM3 | ATGGCGGATCAGCTCACCGACGATCAGATCTCTGAGTTTAA GGAAGCTTTCAGCTTATTCGACAAGGATGGTGATGGTTGCA TTACCACCAAGGAGCTGGGTACTGTGATGCGTTCCCTTGGA CAAAACCCAACCGAAGCAGAGCTTCAAGACATGATCAACGA AGTGGATGCTGATGGTAACGGTACCATTGATTTCCCAGAGT TCTTGAACCTTATGGCTCGTAAGATGAAGGACACCGACTCT GAGGAAGAGCTCAAGGAAGCATTCCGGGTTTTCGACAAGGA CCAGAACGGTTTCATCTCAGCAGCTGAGCTCCGCCATGTGA TGACAAACCTTGGCGAGAAGCTTACTGATGAAGAAGTTGAT GAGATGATCAAGGAAGCTGATGTTGATGGTGATGGTCAGAT TAACtACGAAGAGTTTGTTAAGGTCATGATGGCTAAGTGAC T |
| **2** | *Arabidopsis thaliana* CaM3 | MADQLTDDQISEFKEAFSLFDKDGDCITTKELGTVMRSLG QNPTEAELQDMINEVDADGNGTIDFPEFLNLMARKMKDTDS EEELKEAFRVFDKDQNGFISAAELRHVMTNLGEKLTDEEVD EMIKEADVDGDGQINYEEFVKVMMAK |
| **3** | CaM3 EF-hand1 loop | DKDGDGCITTKE |
| **4** | CaM3 EF-hand2 loop | DADGNGTIDFPE |
| **5** | CaM3 EF-hand3 loop | DKDQNGFISAAE |
| **6** | CaM3 EF-hand4 loop | DVDGDGQINYEE |
| **7** | EF-hand1 loop ΔK2D3 | DGDGCITTKE |
| **8** | EF-hand1 loop ΔK2D3 +C7Y | DGDGYITTKE |
| **9** | EF-hand1 loop ΔK2D3 +C7Y+T10A+K11A | DGDGYITAAE |
| **10** | EF-hand2 loop ΔA2D3 | DGNGTIDFPE |
| **11** | EF-hand2 loop ΔA2D3+T7Y | DGNGYIDFPE |
| **12** | EF-hand2 loop ΔA2D3+N5D | DGDGTIDFPE |
| **13** | EF-hand2 loop ΔA2D3+T7Y+N5D | DGDGYIDFPE |
| **14** | EF-hand3 loop ΔK2D3 | DQNGFISAAE |
| **15** | EF-hand4 loop ΔV2D3 | DGDGQINYEE |
| **16** | CaMΔ | TCC ATG GCG GAT CAG CTC ACC GAC GAT CAG ATC TCT GAG TTT AAG GAA GCT TTC AGC TTA TTC GAC GGT GAT GGT TaC ATT ACC GCC GCG GAG CTG GGT ACT GTG ATG CGT TCC CTT GGA CAA AAC CCA ACC GAA GCA GAG CTT CAA GAC ATG ATC AAC GAA GTG GcT GCT GcT GGT AAC GGT ACC ATT GAT TTC CCA GAG TTC TTG AAC CTT ATG GCT CGT AAG TGA |
| **17** | CaMΔ | SMADQLTDDQISEFKEAFSLFDGDGYITAAELGTVMRSLGQ NPTEAELQDMINEVAAAGNGTIDFPEFLNLMARK |
| **18** | Calmodulin variant from Cameleon biosensor Δ | MADQLTDDQI SEFKEAFSLFDGDGCITTKELGTVMRSLGQN PTEAELQDMINEVDADGNGTIDFPEFLNLMARKMKDTDSEE ELKEAFRVFDKDQNGFI SAAELRHVMTNLGEKLTDEEVDEM IKEADVDGDGQINYEEFVKVMMAK |
| **19** | Calmodulin variant from Cameleon biosensor Δ-S2M | MADQLTDDQISEFKEAFSLFDKDGDGCITTKELGTVMRSLG QNPTEAELQDMINEVAAAGNGTIDFPEFLNLMARKMKDTDS EEELKEAFRVFDKDQNGFISAAELRHVMTNLGEKLTDEEVD EMIKEADVDGDGQINYEEFVKVMMAK |
| **20** | Calmodulin variant (delta sites 1, 2, 3, 4) | MADQLTDDQISEFKEAFSLFDGDGCITTKELGTVMRSLGQN PTEAELQDMINEVDGNGTIDFPEFLNLMARKMKDTDSEEEL KEAFRVFDQNGFISAAELRHVMTNLGEKLTDEEVDEMIKEA DGDGQINYEEFVKVMMAK |
| **21** | M13 | AAACGTCGCTGGAAAAAAACTTTATTGCGGTGAGCGCGGC CAACCGCTTTAAAAAAATTAGCTCGAGCGGCGCGCTG |
| **22** | M13 | KRRWKKNFIAVSAANRFKKISSSGAL |
| **23** | skMLCK | KRRWKKNFIAVSAANRFKKISSSGA |
| **24** | MLCKp | RRKWQKTGHAVRAIGRL |
| **25** | smMLCK | ARRKWQKTGHAVRAIGRLSS |
| **26** | wasp venom | VNWKKIGQHILSV |
| **27** | p21 | KRRQTSMTDFYHSKRRLIFSKRKP |
| **28** | melittin | QQRKRKIWSILAPLGTTLVKLVAGIG |
| **29** | spectrin | KTASPWKSARLMVHTVATFNSIKE |
| **30** | CaMKI | AKSKWKQAFNATAVVRHMRKLQ |
| **31** | CaMKII | LKKFNARRKLKGAILTTMLATRNFS |
| **32** | CaMKK | RFPNGFRKRHGMAKVLILTDLRPIRRV |
| **33** | peptide1 | LKWKKLLKLLKKLLKLG |
| **34** | Cameleon biosensor Δ | TCCATGGTGAGCAAGGGCGAGGAGCTGTTCACCGGGGTGGT GCCCATCCTGGTCGAGCTGGACGGCGACGTAAACGGCCACA GGTTCAGCGTGTCCGGCGAGGGCGAGGGCGATGCCACCTAC GGCAAGCTGACCCTGAAGTTCATCTGCACCACCGGCAAGCT |
| | | GCCCGTGCCCTGGCCCACCCTCGTGACCACCCTGACCTGGG GCGTGCAGTGCTTCAGCCGCTACCCCGACCACATGAAGCAG CACGACTTCTTCAAGTCCGCCATGCCCGAAGGCTACGTCCA GGAGCGTACCATCTTCTTCAAGGACGACGGCAACTACAAGA CCCGCGCCGAGGTGAAGTTCGAGGGCGACACCCTGGTGAAC CGCATCGAGCTGAAGGGCATCGACTTCAAGGAGGACGGCAA CATCCTGGGGCACAAGCTGGAGTACAACTACATCAGCCACA ACGTCTATATCACCGCCGACAAGCAGAAGAACGGCATCAAG GCCCACTTCAAGATCCGCCACAACATCGAGGACGGCAGCGT GCAGCTCGCCGACCACTACCAGCAGAACACCCCCATCGGCG ACGGCCCCGTGCTGCTGCCCGACAACCACTACCTGAGCACC CAGTCCGCCCTGAGCAAAGACCCCAACGAGAAGCGCGATCA CATGGTCCTGCTGGAGTTCGTGACCGCCGCCGAGCTCATGG CGGATCAGTTGACCGACGATCAGATCTCTGAATTTAAGGAA GCCTTCAGCTTATTCGACGGTGATGGTTGCATTACCACCAA GGAACTGGGTACTGTGATGCGTTCCCTGGGCCAAAACCCGA CCGAAGCAGAGCTGCAAGACATGATCAACGAAGTGGATGCG GATGGTAACGGTACCATTGATTTCCCGGAATTCTTGAACCT GATGGCCCGTAAGATGAAAGACACCGACAGCGAGGAAGAGC TGAAAGAAGCCTTCCGCGTTTTCGACAAAGACCAGAACGGT TTCATCAGCGCAGCGGAACTGCGCCATGTGATGACCAACCT GGGCGAAAAACTGACGGATGAAGAAGTTGATGAGATGATCA AAGAAGCGGATGTGGATGGTGATGGTCAGATTAACTACGAA GAGTTTGTTAAGGTGATGATGGCGAAAGGCGGTGGCGGTAG CAAACGTCGCTGGAAAAAAAACTTTATTGCGGTGAGCGCGG CCAACCGCTTTAAAAAAATTAGCTCGAGCGGCGCGCTGGTC GACATGGTGAGCAAGGGCGAGGAGCTGTTCACCGGGGTGGT GCCCATCCTGGTCGAGCTGGACGGCGACGTAAACGGCCACA AGTTCAGCGTGTCCGGCGAGGGCGAGGGCGATGCCACCTAC GGCAAGCTGACCCTGAAGTTCATCTGCACCACCGGCAAGCT GCCCGTGCCCTGGCCCACCCTCGTGACCACCTTCGGCTACG GCGTGCAGTGCTTCGCCCGCTACCCCGACCACATGAAGCAG CACGACTTCTTCAAGTCCGCCATGCCCGAAGGCTACGTCCA GGAGCGCACCATCTTCTTCAAGGACGACGGCAACTACAAGA CCCGCGCCGAGGTGAAGTTCGAGGGCGACACCCTGGTGAAC CGCATCGAGCTGAAGGGCATCGACTTCAAGGAGGACGGCAA CATCCTGGGGCACAAGCTGGAGTACAACTACAACAGCCACA ACGTCTATATCATGGCCGACAAGCAGAAGAACGGCATCAAG GTGAACTTCAAGATCCGCCACAACATCGAGGACGGCAGCGT GCAGCTCGCCGACCACTACCAGCAGAACACCCCCATCGGCG ACGGCCCCGTGCTGCTGCCCGACAACCACTACCTGAGCTAC CAGTCCGCCCTGAGCAAAGACCCCAACGAGAAGCGCGATCA CATGGTCCTGCTGGAGTTCGTGACCGCCGCCGGGATCACTC TCGGCATGGACGAGCTGTACAAGTAA |
| 35 | Cameleon biosensor Δ | SMVSKGEELFTGVVPILVELDGDVNGHRFSVSGEGEGDATY GKLTLKFICTTGKLPVPWPTLVTTLTWGVQCFSRYPDHMKQ HDFFKSAMPEGYVQERTIFFKDDGNYKTRAEVKFEGDTLVN RIELKGIDFKEDGNILGHKLEYNYISHNVYITADKQKNGIK AHFKIRHNIEDGSVQLADHYQQNTPIGDGPVLLPDNHYLST QSALSKDPNEKRDHMVLLEFVTAAELMADQLTDDQISEFKE AFSLFDGDGCITTKELGTVMRSLGQNPTEAELQDMINEVDA DGNGTIDFPEFLNLMARKMKDTDSEEELKEAFRVFDKDQNG FISAAELRHVMTNLGEKLTDEEVDEMIKEADVDGDGQINYE EFVKVMMAKGGGGSKRRWKKNFIAVSAANRFKKISSSGALv dMVSKGEELFTGVVPILVELDGDVNGHKFSVSGEGEGDATY GKLTLKFICTTGKLPVPWPTLVTTFGYGVQCFARYPDHMKQ HDFFKSAMPEGYVQERTIFFKDDGNYKTRAEVKFEGDTLVN RIELKGIDFKEDGNILGHKLEYNYNSHNVYIMADKQKNGIK |
| | | VNFKIRHNIEDGSVQLADHYQQNTPIGDGPVLLPDNHYLSY QSALSKDPNEKRDHMVLLEFVTAAGITLGMDELYK |
| 36 | Cameleon biosensor Δ-S2M | TCCATGGTGAGCAAGGGCGAGGAGCTGTTCACCGGGGTGGT GCCCATCCTGGTCGAGCTGGACGGCGACGTAAACGGCCACA GGTTCAGCGTGTCCGGCGAGGGCGAGGGCGATGCCACCTAC GGCAAGCTGACCCTGAAGTTCATCTGCACCACCGGCAAGCT GCCCGTGCCCTGGCCCACCCTCGTGACCACCCTGACCTGGG GCGTGCAGTGCTTCAGCCGCTACCCCGACCACATGAAGCAG CACGACTTCTTCAAGTCCGCCATGCCCGAAGGCTACGTCCA GGAGCGTACCATCTTCTTCAAGGACGACGGCAACTACAAGA CCCGCGCCGAGGTGAAGTTCGAGGGCGACACCCTGGTGAAC CGCATCGAGCTGAAGGGCATCGACTTCAAGGAGGACGGCAA CATCCTGGGGCACAAGCTGGAGTACAACTACATCAGCCACA ACGTCTATATCACCGCCGACAAGCAGAAGAACGGCATCAAG GCCCACTTCAAGATCCGCCACAACATCGAGGACGGCAGCGT GCAGCTCGCCGACCACTACCAGCAGAACACCCCCATCGGCG ACGGCCCCGTGCTGCTGCCCGACAACCACTACCTGAGCACC CAGTCCGCCCTGAGCAAAGACCCCAACGAGAAGCGCGATCA CATGGTCCTGCTGGAGTTCGTGACCGCCGCCGAGCTCATGG CGGATCAGTTGACCGACGATCAGATCTCTGAATTTAAGGAA GCCTTCAGCTTATTCGACGGTGATGGTTGCATTACCACCAA GGAACTGGGTACTGTGATGCGTTCCCTGGGCCAAAACCCGA CCGAAGCAGAGCTGCAAGACATGATCAACGAAGTGGCTGCG GCTGGTAACGGTACCATTGATTTCCCGGAATTCTTGAACCT GATGGCCCGTAAGATGAAAGACACCGACAGCGAGGAAGAGC TGAAAGAAGCCTTCCGCGTTTTCGACAAAGACCAGAACGGT TTCATCAGCGCAGCGGAACTGCGCCATGTGATGACCAACCT GGGCGAAAAACTGACGGATGAAGAAGTTGATGAGATGATCA AAGAAGCGGATGTGGATGGTGATGGTCAGATTAACTACGAA GAGTTTGTTAAGGTGATGATGGCGAAAGGCGGTGGCGGTAG CAAACGTCGCTGGAAAAAAAACTTTATTGCGGTGAGCGCGG CCAACCGCTTTAAAAAAATTAGCTCGAGCGGCGCGCTGGTC GACATGGTGAGCAAGGGCGAGGAGCTGTTCACCGGGGTGGT GCCCATCCTGGTCGAGCTGGACGGCGACGTAAACGGCCACA AGTTCAGCGTGTCCGGCGAGGGCGAGGGCGATGCCACCTAC GGCAAGCTGACCCTGAAGTTCATCTGCACCACCGGCAAGCT GCCCGTGCCCTGGCCCACCCTCGTGACCACCTTCGGCTACG GCGTGCAGTGCTTCGCCCGCTACCCCGACCACATGAAGCAG CACGACTTCTTCAAGTCCGCCATGCCCGAAGGCTACGTCCA GGAGCGCACCATCTTCTTCAAGGACGACGGCAACTACAAGA CCCGCGCCGAGGTGAAGTTCGAGGGCGACACCCTGGTGAAC CGCATCGAGCTGAAGGGCATCGACTTCAAGGAGGACGGCAA CATCCTGGGGCACAAGCTGGAGTACAACTACAACAGCCACA ACGTCTATATCATGGCCGACAAGCAGAAGAACGGCATCAAG GTGAACTTCAAGATCCGCCACAACATCGAGGACGGCAGCGT GCAGCTCGCCGACCACTACCAGCAGAACACCCCCATCGGCG ACGGCCCCGTGCTGCTGCCCGACAACCACTACCTGAGCTAC CAGTCCGCCCTGAGCAAAGACCCCAACGAGAAGCGCGATCA CATGGTCCTGCTGGAGTTCGTGACCGCCGCCGGGATCACTC TCGGCATGGACGAGCTGTACAAGTAA |
| 37 | Cameleon biosensor Δ-S2M | SMVSKGEELFTGVVPILVELDGDVNGHRFSVSGEGEGDATY GKLTLKFICTTGKLPVPWPTLVTTLTWGVQCFSRYPDHMKQ HDFFKSAMPEGYVQERTIFFKDDGNYKTRAEVKFEGDTLVN RIELKGIDFKEDGNILGHKLEYNYISHNVYITADKQKNGIK AHFKIRHNIEDGSVQLADHYQQNTPIGDGPVLLPDNHYLST QSALSKDPNEKRDHMVLLEFVTAAELMADQLTDDQISEFKE AFSLFDGDGCITTKELGTVMRSLGQNPTEAELQDMINEVAA |
| | | AGNGTIDFPEFLNLMARKMKDTDSEEELKEAFRVFDKDQNG FISAAELRHVMTNLGEKLTDEEVDEMIKEADVDGDGQINYE EFVKVMMAKGGGGSKRRWKKNFIAVSAANRFKKISSSGALv dMVSKGEELFTGVVPILVELDGDVNGHKFSVSGEGEGDATY GKLTLKFICTTGKLPVPWPTLVTTFGYGVQCFARYPDHMKQ HDFFKSAMPEGYVQERTIFFKDDGNYKTRAEVKFEGDTLVN RIELKGIDFKEDGNILGHKLEYNYNSHNVYIMADKQKNGIK VNFKIRHNIEDGSVQLADHYQQNTPIGDGPVLLPDNHYLSY QSALSKDPNEKRDHMVLLEFVTAAGITLGMDELYK |
| 38 | Cameleon biosensor ΔΔΔΔ | SMVSKGEELFTGVVPILVELDGDVNGHRFSVSGEGEGDATY GKLTLKFICTTGKLPVPWPTLVTTLTWGVQCFSRYPDHMKQ HDFFKSAMPEGYVQERTIFFKDDGNYKTRAEVKFEGDTLVN RIELKGIDFKEDGNILGHKLEYNYISHNVYITADKQKNGIK AHFKIRHNIEDGSVQLADHYQQNTPIGDGPVLLPDNHYLST QSALSKDPNEKRDHMVLLEFVTAAELMADQLTDDQISEFKE AFSLFDGDGCITTKELGTVMRSLGQNPTEAELQDMINEVDG NGTIDFPEFLNLMARKMKDTDSEEELKEAFRVFDQNGFISA AELRHVMTNLGEKLTDEEVDEMIKEADGDGQINYEEFVKVM MAKGGGGSKRRWKKNFIAVSAANRFKKISSSGALvdMVSKG EELFTGVVPILVELDGDVNGHKFSVSGEGEGDATYGKLTLK FICTTGKLPVPWPTLVTTFGYGVQCFARYPDHMKQHDFFKS AMPEGYVQERTIFFKDDGNYKTRAEVKFEGDTLVNRIELKG IDFKEDGNILGHKLEYNYNSHNVYIMADKQKNGIKVNFKIR HNIEDGSVQLADHYQQNTPIGDGPVLLPDNHYLSYQSALSK DPNEKRDHMVLLEFVTAAGITLGMDELYK |
| 39 | CaM1 | TCC ATG GCG GAT CAG CTC ACC GAC GAT CAG ATC TCT GAG TTT AAG GAA GCT TTC AGC TTA TTC GAC AAG GAT GGT GAT GGT TaC ATT ACC GCC GCG GAG CTG GGT ACT GTG ATG CGT TCC CTT GGA CAA AAC CCA ACC GAA GCA GAG CTT CAA GAC ATG ATC AAC GAA GTG GcT GCT GcT GGT AAC GGT ACC ATT GAT TTC CCA GAG TTC TTG AAC CTT ATG GCT CGT AAG TGA |
| 40 | CaM1 | SMADQLTDDQISEFKEAFSLFDKDGDGYITAAELGTVMRSL GQNPTEAELQDMINEVAAAGNGTIDFPEFLNLMARK |
| 41 | Primer DGD S | GAAGCTTTCAGCTTATTCGACGGTGATGGTTACATTACCGC CGCG |
| 42 | Primer DGD AS | CGCGGCGGTAATGTAACCATCACCGTCGAATAAGCTGAAAG CTTC |
| 43 | Primer S-TEV-eCFP- *Bam*HI | GAGA GGATCC GAG AAC CTG TAC TTC CAG TCC ATG GTG AGC AAG GGC GAG GAG |
| 44 | Primer AS-eCFP-*Sac*I | TAAA GAGCTC GGCGGCGGTCACGAACTCCAGCA |
| 45 | Primer S-eYFP- *Sal*I | TATA GTCGAC ATG GTG AGC AAG GGC GAG GAG |
| 46 | Primer AS-eYFP-*Hind*III | GGGC AAGCTT TTA CTT GTA CAG CTC GTC CAT GCC G |
| 47 | Cameleon biosensor WT | TCCATGGTGAGCAAGGGCGAGGAGCTGTTCACCGGGGTGGT GCCCATCCTGGTCGAGCTGGACGGCGACGTAAACGGCCACA GGTTCAGCGTGTCCGGCGAGGGCGAGGGCGATGCCACCTAC GGCAAGCTGACCCTGAAGTTCATCTGCACCACCGGCAAGCT GCCCGTGCCCTGGCCCACCCTCGTGACCACCCTGACCTGGG GCGTGCAGTGCTTCAGCCGCTACCCCGACCACATGAAGCAG CACGACTTCTTCAAGTCCGCCATGCCCGAAGGCTACGTCCA GGAGCGTACCATCTTCTTCAAGGACGACGGCAACTACAAGA CCCGCGCCGAGGTGAAGTTCGAGGGCGACACCCTGGTGAAC |
| | | CGCATCGAGCTGAAGGGCATCGACTTCAAGGAGGACGGCAA CATCCTGGGGCACAAGCTGGAGTACAACTACATCAGCCACA ACGTCTATATCACCGCCGACAAGCAGAAGAACGGCATCAAG GCCCACTTCAAGATCCGCCACAACATCGAGGACGGCAGCGT GCAGCTCGCCGACCACTACCAGCAGAACACCCCCATCGGCG ACGGCCCCGTGCTGCTGCCCGACAACCACTACCTGAGCACC CAGTCCGCCCTGAGCAAAGACCCCAACGAGAAGCGCGATCA CATGGTCCTGCTGGAGTTCGTGACCGCCGCCGAGCTCATGG CGGATCAGTTGACCGACGATCAGATCTCTGAATTTAAGGAA GCCTTCAGCTTATTCGACAAAGATGGTGATGGTTGCATTAC CACCAAGGAACTGGGTACTGTGATGCGTTCCCTGGGCCAAA ACCCGACCGAAGCAGAGCTGCAAGACATGATCAACGAAGTG GATGCGGATGGTAACGGTACCATTGATTTCCCGGAATTCTT GAACCTGATGGCCCGTAAGATGAAAGACACCGACAGCGAGG AAGAGCTGAAAGAAGCCTTCCGCGTTTTCGACAAAGACCAG AACGGTTTCATCAGCGCAGCGGAACTGCGCCATGTGATGAC CAACCTGGGCGAAAAACTGACGGATGAAGAAGTTGATGAGA TGATCAAAGAAGCGGATGTGGATGGTGATGGTCAGATTAAC TACGAAGAGTTTGTTAAGGTGATGATGGCGAAAGGCGGTGG CGGTAGCAAACGTCGCTGGAAAAAAAACTTTATTGCGGTGA GCGCGGCCAACCGCTTTAAAAAAATTAGCTCGAGCGGCGCG CTGGTCGACATGGTGAGCAAGGGCGAGGAGCTGTTCACCGG GGTGGTGCCCATCCTGGTCGAGCTGGACGGCGACGTAAACG GCCACAAGTTCAGCGTGTCCGGCGAGGGCGAGGGCGATGCC ACCTACGGCAAGCTGACCCTGAAGTTCATCTGCACCACCGG CAAGCTGCCCGTGCCCTGGCCCACCCTCGTGACCACCTTCG GCTACGGCGTGCAGTGCTTCGCCCGCTACCCCGACCACATG AAGCAGCACGACTTCTTCAAGTCCGCCATGCCCGAAGGCTA CGTCCAGGAGCGCACCATCTTCTTCAAGGACGACGGCAACT ACAAGACCCGCGCCGAGGTGAAGTTCGAGGGCGACACCCTG GTGAACCGCATCGAGCTGAAGGGCATCGACTTCAAGGAGGA CGGCAACATCCTGGGGCACAAGCTGGAGTACAACTACAACA GCCACAACGTCTATATCATGGCCGACAAGCAGAAGAACGGC ATCAAGGTGAACTTCAAGATCCGCCACAACATCGAGGACGG CAGCGTGCAGCTCGCCGACCACTACCAGCAGAACACCCCCA TCGGCGACGGCCCCGTGCTGCTGCCCGACAACCACTACCTG AGCTACCAGTCCGCCCTGAGCAAAGACCCCAACGAGAAGCG CGATCACATGGTCCTGCTGGAGTTCGTGACCGCCGCCGGGA TCACTCTCGGCATGGACGAGCTGTACAAGTAA |
| 48 | Cameleon biosensor WT | SMVSKGEELFTGVVPILVELDGDVNGHRFSVSGEGEGDATY GKLTLKFICTTGKLPVPWPTLVTTLTWGVQCFSRYPDHMKQ HDFFKSAMPEGYVQERTIFFKDDGNYKTRAEVKFEGDTLVN RIELKGIDFKEDGNILGHKLEYNYISHNVYITADKQKNGIK AHFKIRHNIEDGSVQLADHYQQNTPIGDGPVLLPDNHYLST QSALSKDPNEKRDHMVLLEFVTAAELMADQLTDDQISEFKE AFSLFDKDGDGCITTKELGTVMRSLGQNPTEAELQDMINEV DADGNGTIDFPEFLNLMARKMKDTDSEEELKEAFRVFDKDQ NGFISAAELRHVMTNLGEKLTDEEVDEMIKEADVDGDGQIN YEEFVKVMMAKGGGGSKRRWKKNFIAVSAANRFKKISSSGA LvdMVSKGEELFTGVVPILVELDGDVNGHKFSVSGEGEGDA TYGKLTLKFICTTGKLPVPWPTLVTTFGYGVQCFARYPDHM KQHDFFKSAMPEGYVQERTIFFKDDGNYKTRAEVKFEGDTL VNRIELKGIDFKEDGNILGHKLEYNYNSHNVYIMADKQKNG IKVNFKIRHNIEDGSVQLADHYQQNTPIGDGPVLLPDNHYL SYQSALSKDPNEKRDHMVLLEFVTAAGITLGMDELYK |
| 49 | Primer S-Δ | GGAAGCCTTCAGCTTATTCGACGGTGATGGTTGCATTACC |
| 50 | Primer AS-Δ | GGTAATGCAACCATCACCGTCGAATAAGCTGAAGGCTTCC |
| 51 | Primer S-S2M | GGT ACC GTT ACC AGC AGC AGC CAC TTC GTT |
| | | GAT C |
| 52 | Primer AS-S2M | GAT CAA CGA AGT GGC TGC TGC TGG TAA CGG TAC C |
| 53 | Cameleon biosensor WT-S2M | TCCATGGTGAGCAAGGGCGAGGAGCTGTTCACCGGGGTGGT GCCCATCCTGGTCGAGCTGGACGGCGACGTAAACGGCCACA GGTTCAGCGTGTCCGGCGAGGGCGAGGGCGATGCCACCTAC GGCAAGCTGACCCTGAAGTTCATCTGCACCACCGGCAAGCT GCCCGTGCCCTGGCCCACCCTCGTGACCACCCTGACCTGGG GCGTGCAGTGCTTCAGCCGCTACCCCGACCACATGAAGCAG CACGACTTCTTCAAGTCCGCCATGCCCGAAGGCTACGTCCA GGAGCGTACCATCTTCTTCAAGGACGACGGCAACTACAAGA CCCGCGCCGAGGTGAAGTTCGAGGGCGACACCCTGGTGAAC CGCATCGAGCTGAAGGGCATCGACTTCAAGGAGGACGGCAA CATCCTGGGGCACAAGCTGGAGTACAACTACATCAGCCACA ACGTCTATATCACCGCCGACAAGCAGAAGAACGGCATCAAG GCCCACTTCAAGATCCGCCACAACATCGAGGACGGCAGCGT GCAGCTCGCCGACCACTACCAGCAGAACACCCCCATCGGCG ACGGCCCCGTGCTGCTGCCCGACAACCACTACCTGAGCACC CAGTCCGCCCTGAGCAAAGACCCCAACGAGAAGCGCGATCA CATGGTCCTGCTGGAGTTCGTGACCGCCGCCGAGCTCATGG CGGATCAGTTGACCGACGATCAGATCTCTGAATTTAAGGAA GCCTTCAGCTTATTCGACAAAGATGGTGATGGTTGCATTAC CACCAAGGAACTGGGTACTGTGATGCGTTCCCTGGGCCAAA ACCCGACCGAAGCAGAGCTGCAAGACATGATCAACGAAGTG GCTGCGGCTGGTAACGGTACCATTGATTTCCCGGAATTCTT GAACCTGATGGCCCGTAAGATGAAAGACACCGACAGCGAGG AAGAGCTGAAAGAAGCCTTCCGCGTTTTCGACAAAGACCAG AACGGTTTCATCAGCGCAGCGGAACTGCGCCATGTGATGAC CAACCTGGGCGAAAAACTGACGGATGAAGAAGTTGATGAGA TGATCAAAGAAGCGGATGTGGATGGTGATGGTCAGATTAAC TACGAAGAGTTTGTTAAGGTGATGATGGCGAAAGGCGGTGG CGGTAGCAAACGTCGCTGGAAAAAAAACTTTATTGCGGTGA GCGCGGCCAACCGCTTTAAAAAAATTAGCTCGAGCGGCGCG CTGGTCGACATGGTGAGCAAGGGCGAGGAGCTGTTCACCGG GGTGGTGCCCATCCTGGTCGAGCTGGACGGCGACGTAAACG GCCACAAGTTCAGCGTGTCCGGCGAGGGCGAGGGCGATGCC ACCTACGGCAAGCTGACCCTGAAGTTCATCTGCACCACCGG CAAGCTGCCCGTGCCCTGGCCCACCCTCGTGACCACCTTCG GCTACGGCGTGCAGTGCTTCGCCCGCTACCCCGACCACATG AAGCAGCACGACTTCTTCAAGTCCGCCATGCCCGAAGGCTA CGTCCAGGAGCGCACCATCTTCTTCAAGGACGACGGCAACT ACAAGACCCGCGCCGAGGTGAAGTTCGAGGGCGACACCCTG GTGAACCGCATCGAGCTGAAGGGCATCGACTTCAAGGAGGA CGGCAACATCCTGGGGCACAAGCTGGAGTACAACTACAACA GCCACAACGTCTATATCATGGCCGACAAGCAGAAGAACGGC ATCAAGGTGAACTTCAAGATCCGCCACAACATCGAGGACGG CAGCGTGCAGCTCGCCGACCACTACCAGCAGAACACCCCCA TCGGCGACGGCCCCGTGCTGCTGCCCGACAACCACTACCTG AGCTACCAGTCCGCCCTGAGCAAAGACCCCAACGAGAAGCG CGATCACATGGTCCTGCTGGAGTTCGTGACCGCCGCCGGGA TCACTCTCGGCATGGACGAGCTGTACAAGTAA |
| 54 | Cameleon biosensor WT-S2M | SMVSKGEELFTGVVPILVELDGDVNGHRFSVSGEGEGDATY GKLTLKFICTTGKLPVPWPTLVTTLTWGVQCFSRYPDHMKQ HDFFKSAMPEGYVQERTIFFKDDGNYKTRAEVKFEGDTLVN RIELKGIDFKEDGNILGHKLEYNYISHNVYITADKQKNGIK AHFKIRHNIEDGSVQLADHYQQNTPIGDGPVLLPDNHYLST QSALSKDPNEKRDHMVLLEFVTAAELMADQLTDDQISEFKE AFSLFDKDGDGCITTKELGTVMRSLGQNPTEAELQDMINEV |
| | | AAAGNGTIDFPEFLNLMARKMKDTDSEEELKEAFRVFDKDQ NGFISAAELRHVMTNLGEKLTDEEVDEMIKEADVDGDGQIN YEEFVKVMMAKGGGGSKRRWKKNFIAVSAANRFKKISSSGA LvdMVSKGEELFTGVVPILVELDGDVNGHKFSVSGEGEGDA TYGKLTLKFICTTGKLPVPWPTLVTTFGYGVQCFARYPDHM KQHDFFKSAMPEGYVQERTIFFKDDGNYKTRAEVKFEGDTL VNRIELKGIDFKEDGNILGHKLEYNYNSHNVYIMADKQKNG IKVNFKIRHNIEDGSVQLADHYQQNTPIGDGPVLLPDNHYL SYQSALSKDPNEKRDHMVLLEFVTAAGITLGMDELYK |

## Claims

1. A polypeptide comprising at least one helix-loop-helix calcium-binding (EF-hand) motif which comprises a deletion of at least one amino acid in the 12-amino-acid calcium-binding loop sequence, and wherein said polypeptide binds uranyl.

2. The polypeptide of claim 1, which comprises a deletion of at least one of the calcium-ligating residues in positions 1, 3, and/or 5 of said calcium-binding loop sequence.

3. The polypeptide of claim 2, which comprises a deletion of at least one of the following pairs of residues: positions 1 and 2, 2 and 3, 3 and 4, 4 and 5 and 5 and 6 of said calcium-binding loop sequence.

4. The polypeptide of any one of claims 1 to 3, wherein said EF-hand motif(s) are derived from signaling EF-hand protein(s) of the calmodulin superfamily selected from the group consisting of calmodulin and troponin C.

5. The polypeptide of any one of claims 1 to 4, which comprises two or four EF-hand motifs, wherein at least one EF-hand motif comprises said deletion in the calcium-binding loop sequence and the other EF-hand motif(s) comprise said deletion or not.

6. The polypeptide of claim 4 or 5, which is a calmodulin domain 1 variant comprising two EF-hand motifs, respectively from the EF-hand1 and the EF-hand2 of the same calmodulin protein.

7. The polypeptide of claim 6, which comprises or consists of an amino acid sequence selected from the group consisting of the sequences SEQ ID NO: 17 to 20.

8. A fusion protein comprising a polypeptide according to any one of claims 1 to 7 fused to another protein moiety.

9. The fusion protein of claim 8, which is a cameleon protein comprising tandem fusions of (1) a polypeptide of claim 5 which comprises four EF-hand motifs (2) a EF-hand protein binding-peptide that binds a complex between the polypeptide in (1) and calcium, (3) a fluorescence-donor protein, and (4) a fluorescence-acceptor protein.

10. The fusion protein of claim 9, which comprises or consists of a sequence selected from the group consisting of the sequences SEQ ID NO: 35, 37 and 38..

11. The polypeptide of any one of claims 1 to 7 or the fusion protein of any one of claims claim 8 to 10, which is immobilized onto a solid support.

12. A polynucleotide encoding the polypeptide of any one of claims 1 to 7 or the fusion protein of any one of claims 8 to 10.

13. A host cell modified with the polynucleotide of claim 12.

14. A non-human transgenic organism modified with the polynucleotide of claim 12.

15. Use of the polypeptide of any one of claims 1 to 7, the fusion protein of any one of claims 8 to 10, the host cell of claim 13 or the non-human transgenic organism of claim 14 as uranyl chelating agent, for the detection of uranium contamination *in vitro* or for the decontamination and/or the bioremediation of uranium contamination in an environment.
